# EUROPEAN PATENT APPLICATION

(11) **EP 4 736 738 A1**
(43) Date of publication of application: **06.05.2026**
(21) Application number: 24210112.9
(22) Date of filing: 31.10.2024
(51) Int. Cl.: A61B 3/10, A61B 3/12, A61B 3/11

(54) **AN OCT APPARATUS FOR OPTORETINOGRAPHY**

(71) Applicant: Optos plc, Dunfermline, Scotland KY11 8GR (GB)
(72) Inventor: Rycroft, Ewan, Dunfermline, Scotland, KY11 8GR (GB); Preciado, Miguel, Dunfermline, Scotland, KY11 8GR (GB); Normand, Margaret, Dunfermline, Scotland, KY11 8GR (GB)
(74) Representative: Hoffmann Eitle

(57) **Abstract**

An OCT apparatus for acquiring optoretinography data, comprising: an optical system which applies an optical stimulus confined to a portion of a retina of the eye whose location is controllable; an OCT imaging system which acquires OCT data from the retina; and a controller which: acquires a duration of a physiological response of the retina in the optoretinography data to be acquired; acquires target locations on the retina and uses these to control the optical system to apply the optical stimulus to respective first portions of the retina at the respective target locations; controls the OCT imaging system to acquire, for each first portion, respective OCT data of the respective second portion of the retina over the duration indicated by the first indicator, which second portion is stimulated by the optical stimulus applied to the first portion; and generates the ORG data based on the acquired OCT data.

## Description

### [Field]

Example aspects herein generally relate to the field of optical coherence tomography (OCT) imaging systems and, in particular, to OCT imaging systems for acquiring optoretinography (ORG) data indicative of a physiological response of a retina of an eye of a subject to an optical stimulus.

### [Background]

Optical coherence tomography (OCT) is an imaging technique based on low-coherence interferometry, which is widely used to acquire high-resolution two- and three-dimensional images of optical scattering media, such as biological tissue.

OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between light in the reference arm and light in the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back-reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

Modern FD-OCT imaging systems are often phase-stable. For example, SD-OCT imaging systems are inherently phase-stable due to the simultaneous acquisition of all the spectral sampling points with a line-scan camera. SS-OCT imaging systems may also be phase-stable by employing phase-stabilization techniques well-known to those versed in the art.

OCT imaging systems can also be classified as being point-scan (also known as "point detection" or "scanning point"), line-scan or full-field, depending on how the imaging system is configured to acquire OCT data at locations on the imaging target. A point-scan OCT imaging system acquires OCT data by scanning a focused sample beam across the surface of the imaging target, typically along a single line (which may be straight, or alternatively curved so as to define a circle or a spiral, for example) or along a set of (usually substantially parallel) lines on the surface of the imaging target, and acquiring an axial depth profile (A-scan) for each of a plurality of points along the line(s), one single point at a time, to build up OCT data comprising a one- or two-dimensional array of A-scans representing a two-dimensional (i.e. a B-scan) or three-dimensional (i.e. a C-scan or volumetric scan) reflectance profile of the sample.

A line-scan OCT imaging system acquires OCT data by scanning a focused line of light across the surface of the imaging target. Measured reflectance from the imaging target is used to generate OCT data comprising a two-dimensional reflectance profile (i.e. a B-scan) of the sample. By scanning the focused line of light across a plurality of locations on the imaging target, OCT data comprising a three-dimensional reflectance profile (i.e. a C-scan or volumetric scan) of the sample can be obtained. Typically, the focused line of light is straight and is scanned in a direction perpendicular to it, although in some instances it may be curved with the scanning direction adjusted accordingly. A full-field OCT imaging system acquires OCT data by projecting a beam of light onto the imaging target to acquire OCT data comprising a three-dimensional reflectance profile (i.e. a C-scan or volumetric scan) of the sample.

Optoretinography (ORG) generally refers to the detection of a physiological response of a retina of an eye to an optical stimulus (i.e. light-induced functional activity of the retina). ORG techniques include the non-invasive optical imaging of this physiological response of the retina. For example, OCT imaging systems can be used to image retinal neurons thought to be exhibiting a change in dimension (size) in response to excitation by the optical stimulus. These changes in dimension have been shown to be detectable by OCT imaging systems and are typically changes in length of the outer segments of cone photoreceptors or rod photoreceptors in the retina that are detected by measuring the change in axial depth of the inner-outer segment (IS/OS) junction and of the cone outer segment tip (COST) of the cone photoreceptors, or the change in axial depth of the IS/OS junction and of the rod outer segment tip (ROST) of rod photoreceptors, respectively. However, the detection of changes in dimension of other retinal neurons has also been demonstrated with OCT imaging systems such as, for example, those of retinal ganglion cells. The ganglion cell layer/inner plexiform layer (GCL/IPL) may also produce a measurable change in thickness when stimulated. The GCL and IPL tend to provide much weaker reflections that are nevertheless detectable, particularly where steps are taken to suppress motion artefacts (see, for example "Simultaneous functional imaging of neuronal and photoreceptor layers in living human retina" by C. Pfäffle et al., Optic Letters, Vol. 44, No. 23, pages 5671-5674 (1 December 2019)).

Existing ORG techniques using an OCT imaging system to acquire ORG data indicative of the physiological response of a portion of the retina to the optical stimulus typically rely on a preliminary period of dark adaptation of the retina to ensure that the retinal neurons are in an unstimulated state, with the optical stimulus subsequently being applied to the retina within the full field of view of the OCT imaging system (i.e. the region of the retina over which the OCT imaging system is operable to acquire OCT data whilst the eye is fixated on a fixation target). After acquiring ORG data at a first location on the retina, the retina must undergo a further period of dark adaptation before respective ORG data can be acquired at each additional location on the retina, as the whole of the retina within the field of view of the OCT imaging system is stimulated during the acquisition of the ORG data at each location.

However, as the period for dark adaptation is typically of the order of several minutes (e.g. approximately 5 minutes) or more, repeating this dark adaptation period substantially increases the time taken to acquire ORG data from different portions of the retina. This can be both tiresome for the patient and reduce the rate at which ORG data can be acquired from patients, thus limiting the rate at which a clinician can examine patients.

### [Summary]

There is provided, in accordance with a first example aspect herein, an OCT apparatus arranged to acquire optoretinography (ORG) data that is indicative of a physiological response of a retina of an eye of a subject to an optical stimulus, the OCT apparatus comprising: an optical system operable to apply the optical stimulus to the retina such that an illumination of the retina by the optical stimulus is confined to a portion of the retina, the optical system being controllable to vary a location on the retina at which the optical stimulus is to be applied; an OCT imaging system operable to acquire OCT data by imaging a portion of the retina of the eye; and a controller. The controller is arranged to: acquire a first indicator which is indicative of a duration of the physiological response indicated by ORG data that is to be acquired by the OCT apparatus; acquire a number, N, of second indicators (N being an integer greater than or equal to 2), each second indicator being indicative of a respective target location on the retina at which the optical stimulus is to be applied by the optical system, wherein N is dependent on the first indicator such that N decreases as the duration indicated by the first indicator increases; use the second indicators to control the optical system to apply the optical stimulus to respective first portions of the retina at the respective target locations; control the OCT imaging system to acquire, for each of the first portions of the retina, respective OCT data of a respective second portion of N second portions of the retina over the duration indicated by the first indicator, wherein at least a part of the respective second portion of the retina is disposed in relation to the respective first portion of the retina so as to be stimulated by the applied optical stimulus during acquisition of at least some of the respective OCT data; and process the respective OCT data of each second portion of the retina to generate respective ORG data indicative of a respective physiological response of the second portion of the retina to the optical stimulus applied to the corresponding first portion of the retina.

In some example embodiments, the controller is arranged to acquire the first indicator by selecting a value from a group of values comprising: a first value indicative of a duration less than 20 ms of the physiological response indicated by ORG data that is to be acquired by the OCT apparatus; and a second value indicative of a duration greater than 20 ms of the physiological response indicated by ORG data that is to be acquired by the OCT apparatus.

In these or other example embodiments, the N second indicators may be indicative of respective target locations on the retina that are one of: distributed around a circle centred on a fovea of the eye, at which target locations the optical stimulus is to be applied by the optical system; located within respective grid cells of an Early Treatment Diabetic Retinopathy Study (ETDRS) grid centred on a fovea of the eye, at which target locations the optical stimulus is to be applied by the optical system; and distributed along a straight line passing through a fovea of the eye, at which target locations the optical stimulus is to be applied by the optical system.

In any of the above example embodiments, the optical system may comprise a light source arranged to generate light which provides the optical stimulus, and one or more scanning elements arranged to direct the light to the retina, and the controller may be arranged to use the N second indicators to control the one or more scanning elements to direct the light to each of the first portions of the retina which is at the respective target location.

In such example embodiments, the OCT imaging system may comprise: an interferometer having a sample arm and a reference arm; and a detector arranged to detect an interference between sample OCT light propagating along the sample arm after having been scattered from the retina, and reference OCT light propagating along the reference arm, wherein at least one of the one or more scanning elements is further arranged to direct the sample OCT light toward each of the N second portions of the retina, and the sample OCT light scattered from each of the second portions of the retina toward the detector. Alternatively, in those example embodiments, the OCT imaging system may comprise: an interferometer having a sample arm and a reference arm; one or more scanning elements; and a detector arranged to detect an interference between sample OCT light propagating along the sample arm after having been scattered from the retina, and reference OCT light propagating along the reference arm, wherein the one or more scanning elements are arranged to direct the sample OCT light toward each of the N second portions of the retina, and the sample OCT light scattered from each of the N second portions of the retina toward the detector, and wherein the one or more scanning elements of the optical system are different from the one or more scanning elements of the OCT imaging system. In this case, the controller may be arranged to use the N second indicators to control the one or more scanning elements of the optical system independently from the one or more scanning elements of the OCT imaging system.

In any of the above example embodiments, the controller may store a third indicator which is indicative of a period of time over which the optical system is to apply the optical stimulus to respective first portions of the retina and the OCT imaging system is to acquire, for each of the first portions of the retina, the respective OCT data of the respective second portion of the retina over the duration indicated by the first indicator, and the controller may be arranged to determine, based on the first indicator and the third indicator, the number, N, of second indicators to be acquired such that, within the period of time indicated by the third indicator, uses the N second indicators to control the optical system to apply the optical stimuli to the respective first portions of the retina, and the controller controls the OCT imaging system to acquire the respective OCT data for each of the first portions of the retina. The controller may be arranged to update the third indicator based on a size of the pupil of the eye such that the period of time indicated by the third indicator increases as the size of the pupil increases. In some example embodiments, the period of time indicated by the third indicator does not exceed 300 ms.

In the foregoing, each first portion of the retina may be smaller than a region of the retina over which the OCT imaging system is operable to acquire OCT data.

There is provided, in accordance with a second example aspect herein, a computerimplemented method of controlling an OCT apparatus to acquire optoretinography, ORG, data that is indicative of a physiological response of a retina of an eye to an optical stimulus, the OCT apparatus comprising: an optical system operable to apply the optical stimulus to the retina such that an illumination of the retina by the optical stimulus is confined to a portion of the retina, the optical system being controllable to vary a location on the retina at which the optical stimulus is to be applied; and an OCT imaging system operable to acquire OCT data by imaging a portion of the retina of the eye. The method comprises: acquiring a first indicator which is indicative of a duration of the physiological response indicated by ORG data that is to be acquired by the OCT apparatus; acquiring a number, N, of second indicators, each second indicator being indicative of a respective target location on the retina at which the optical stimulus is to be applied by the optical system, wherein N is dependent on the first indicator such that N decreases as the duration indicated by the first indicator increases; using the second indicators to control the optical system to apply the optical stimulus to respective first portions of the retina at the respective target locations; controlling the OCT imaging system to acquire, for each of the first portions of the retina, respective OCT data of a respective second portion of N second portions of the retina over the duration indicated by the first indicator, wherein at least a part of the respective second portion of the retina is disposed in relation to the respective first portion of the retina so as to be stimulated by the applied optical stimulus during acquisition of at least some of the respective OCT data; and processing the respective OCT data of each second portion of the retina to generate respective ORG data indicative of a respective physiological response of the second portion of the retina to the optical stimulus applied to the corresponding first portion of the retina.

In some example embodiments, the first indicator may be acquired by selecting a value from a group of values comprising: a first value indicative of a duration less than 20 ms of the physiological response indicated by ORG data that is to be acquired by the OCT apparatus; and a second value indicative of a duration greater than 20 ms of the physiological response indicated by ORG data that is to be acquired by the OCT apparatus. Additionally or alternatively, the method may further comprise: storing a third indicator which is indicative of a period of time over which the optical system is to apply the optical stimulus to respective first portions of the retina and the OCT imaging system is to acquire, for each of the first portions of the retina, the respective OCT data of the respective second portion of the retina over the duration indicated by the first indicator; and determining, based on the first indicator and the third indicator, the number, N, of second indicators to be acquired such that, within the period of time indicated by the third indicator, the N second indicators are used to control the optical system to apply the optical stimulus to the respective first portions of the retina, and the OCT imaging system is controlled to acquire the respective OCT data for each of the first portions of the retina.

There is provided, in accordance with a third example aspect herein, a computer program comprising computer-readable instructions that, when executed by a processor which is arranged to control the OCT apparatus according to the first example aspect or any of its example embodiments set out above, cause the processor to control the OCT apparatus in accordance with the method of the second example aspect or any of its example embodiments set out above. The computer program may be stored on a non-transitory computer-readable storage medium, in accordance with a fourth example aspect herein, or it may be carried by a signal.

### [Brief Description of the Drawings]

Example embodimentswill now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of an FD-OCT apparatus 100 according to an example embodiment herein.
Figure 2 is a schematic front view of a display device 200 of the FD-apparatus 100, which displays a graphic 210 as an example of a fixation target for fixing a gaze direction of an eye.
Figure 3A is a schematic illustration of an optical system 300 and an FD-OCT imaging system 320 according to the example embodiment herein.
Figure 3B is a schematic illustration of an example optical system 330 and an example FD-OCT imaging system 331 that share an example scanning system 332, which includes lens-based optics instead of the curved mirrors 313 and 315 in Figure 3A.
Figure 3C is a schematic illustration of an optical system 340 and an FD-OCT imaging system 341 according to an alternative implementation of the example embodiment herein.
Figure 3D is a schematic illustration of an example optical system 350 and an example FD-OCT imaging system 351 that share an example scanning system 352, which includes lens-based optics instead of the curved mirrors 313 and 315 in Figure 3C.
Figure 3E is a schematic illustration of an optical system 360 and an FD-OCT imaging system 361 according to a further alternative implementation of the example embodiment.
Figure 3F is a schematic illustration of an example optical system 370 and an example FD-OCT imaging system 371 that share an example scanning system 372 using a two-dimensional scanner 373 instead of the third scanning element 363 and the fourth scanning element 364 in Figure 3E.
Figure 4 is a schematic illustration of a programmable signal processing hardware, which may be configured to perform the functions of the controller 140 described herein.
Figure 5A is a flow diagram illustrating a process by which controller 140 generates ORG data 150 according to the example embodiment herein.
Figure 5B is a flow diagram illustrating an optional process by which the number, N, of the second indicators described herein may be determined by the controller 140.
Figures 6A, 6B and 6C are schematic illustrations of example first portions and second portions of the retina in the performance of processes S55 and S56 in Figure 5A using the optical system 300 and the FD-OCT imaging system 320 of the example embodiment.
Figures 6D and 6E are schematic illustrations of example first portions and second portions of the retina in the performance of processes S55 and S56 in Figure 5A using the optical system 360 and the FD-OCT imaging system 361 in Figure 3E.
Figure 7 is a schematic illustration of a first portion and a second portion in the performance of processes S55 and S56 in Figure 5A using the optical system 340 and the FD-OCT imaging system 341 in Figure 3C.
Figure 8A shows an example plurality of first portions 801 to 807 and an example plurality of second portions 811 to 817 in the performance of processes S55 and S56 in Figure 5A during an ORG data capture session.
Figure 8B shows an example plurality of first portions 821 to 824 and an example plurality of second portions 825 to 824 in the performance of processes S55 and S56 in Figure 5A during an ORG data capture session.
Figure 8C shows an example plurality of first portions 831 to 834 and an example plurality of second portions 835 to 838 in the performance of processes S55 and S56 in Figure 5A during an ORG data capture session.
Figure 8D shows an example plurality of first portions 841 to 844 and an example plurality of second portions 845 to 848 in the performance of processes S55 and S56 in Figure 5A during and ORG data capture session.
Figure 8E shows an example plurality of first portions 851, 852, 855 and 856, and an example plurality of second portions 853, 854, 857 and 858, in the performance of processes S55 and S56 in Figure 5A during an ORG data capture session.
Figure 9 shows an example first portion 901 and an example second portion 902 in the performance of processes S55 and S56 in Figure 5A during an ORG data capture session.
Figure 10 shows an example first portion 1001 and an example second portion 1002 in the performance of processes S55 and S56 in Figure 5A during an ORG data capture session.
Figure 11 is a schematic illustration of a schedule for successively acquiring sets of circular B-scans of the kind described herein with reference to Figure 6E, at three different locations on the retina as shown in Figure 12, to acquire ORG data relating to the fast response and initial part of the slow response of the retina shown in the inset.
Figure 12 is a schematic illustration of an arrangement, along a circle centred on the fovea, of example first portions and second portions of the retina that are illuminated in the performance of processes S55 and S56 in Figure 5A, using the optical system 360 and the FD-OCT imaging system 361 in Figure 3E and in accordance with the schedule in Figure 11.
Figure 13A is a schematic illustration of an alternative arrangement, along a straight line passing through the fovea, of example first portions and second portions of the retina that are illuminated in the performance of processes S55 and S56 in Figure 5A, using the optical system 360 and the FD-OCT imaging system 361 in Figure 3E and in accordance with the schedule in Figure 11.
Figure 13B is a schematic illustration of an example ORG response plotted as a function of eccentricity, which may be acquired using a linear arrangement of first and second portions of the kind illustrated in Figure 13A.
Figure 14 is a schematic illustration of a schedule for successively acquiring sets of circular B-scans of the kind described herein with reference to Figure 6E, at different locations on the retina, to acquire ORG data relating only to the fast response of the retina shown in the inset.
Figure 15 is a schematic illustration of 21 example second portions of the retina scanned in the performance of process S56 in Figure 5A, using the FD-OCT imaging system 361 in Figure 3E and a schedule for successively acquiring 21 sets of circular B-scans that are of the kind described herein with reference to Figure 6E.
Figure 16 is a schematic illustration of an alternative arrangement of 9 example second portions of the retina, in corresponding portions of an Early Treatment Diabetic Retinopathy Study (ETDRS) grid, that are scanned in the performance of process S56 in Figure 5A using the FD-OCT imaging system 361 of Figure 3E and in accordance with a schedule for successively acquiring 9 sets of circular B-scans that are of the kind described herein with reference to Figure 6E.
Figure 17 is a schematic illustration of a rotatable wedged prism, which may be controlled to set a location on the retina of the eye 160 at which an optical stimulus is to be applied.
Figure 18 is a schematic illustration of a Risley prism scanner, which may be controlled to set a location on the retina of the eye 160 at which an optical stimulus is to be applied.
Figure 19 is a schematic illustration of an alternative optical arrangement, comprising an optical fibre and one or more lenses, which may be used to set a location on the retina of the eye 160 at which an optical stimulus is to be applied.
Figure 20 is a schematic illustration of a further alternative optical arrangement, comprising a dynamic amplitude mask and a beam steering mechanism, which may be used to set a location on the retina of the eye 160 at which an optical stimulus is to be applied.

### [Detailed Description of Example Embodiments]

In view of the above-described problems with using repeated dark adaptation periods to acquire ORG data at multiple locations on the retina, the present inventors have devised an OCT apparatus arranged to acquire optoretinography (ORG) data that is indicative of a physiological response of a retina of an eye to an optical stimulus, the OCT imaging apparatus comprising an optical system operable to apply the optical stimulus to the retina such that an illumination of the retina by the optical stimulus is confined to a portion of the retina and controllable to vary a location on the retina at which the optical stimulus is to be applied. The OCT apparatus may, as in the example embodiments described below, be a Fourier-domain OCT (FD-OCT) apparatus.

The optical stimulus may thus be applied to the localised portion of the retina (without being applied to a surrounding region of the retina) and may be applied to different portions of the retina at different locations on the retina within the field of view of the OCT imaging apparatus, by controlling the optical system. Accordingly, after a single period of dark adaptation, the optical stimulus may stimulate multiple different portions of the retina, without the optical stimulus applied to any of the portions stimulating any other of the portions of the retina. This may allow for accurate, comparable ORG data to be acquired at each of the locations of the portions without the need for further dark adaptation periods or for repeated patient alignment, which may make the acquisition of the ORG data much easier to automate and significantly reduce the time taken to acquire the ORG data. The faster acquisition of the ORG data may improve the comfort of the patient by reducing the time the patient spends in front of the OCT apparatus, consequently reducing the patient's tendency to move and thus degrade the quality of the ORG data. In addition, the rate at which patients can be imaged using the OCT apparatus may be increased, thus improving the rate at which a clinician may assess patients using the OCT apparatus.

Further, a less powerful light source for generating the optical stimulus may be employed than in the conventional case where an optical stimulus is applied to the full field of view of the OCT imaging system, which may be more comfortable for the patient. By confining the optical stimulus to a localised portion of the retina, a sufficiently high irradiance of the portion for ORG may also be achieved despite the use of the less powerful light source.

Furthermore, the inventors have recognised that the tendency of the pupil of the eye to contract in response to applied optical stimuli, and involuntary movements of the subject that inevitably occur, lead to a growing risk of the optical stimuli from the optical system and/or the OCT light being clipped by the pupil as the acquisition of ORG data progresses. The inventors have further recognised that these factors cause the desired measurement timescale for the retinal response to limit the amount of OCT data that can be acquired before the quality of the ORG data becomes degraded by the clipping. To reduce the risk of such degradation of the acquired ORG data, the inventors have recognised that the number, N, of target locations where the optical stimuli are applied needs to vary in dependence on the desired measurement timescale for the retinal response, such that N decreases as the measurement timescale increases. Thus, the longer the measurement timescale, the fewer locations on the retina should be targeted for OCT measurements.

The number N preferably varies with the desired measurement timescale such that, within the time window imposed by the factors noted above, for example, the optical system applies the optical stimulus to respective first portions of the retina, and respective OCT data is acquired for each of the first portions of the retina over the desired measurement timescale. In this case, all of the OCT data can be acquired, from N second portions of the retina that are stimulated by the application of the stimulus to the corresponding first portions, within a time interval which is smaller enough to reduce or avoid the clipping of the optical stimulus and/or the OCT light by the pupil of the eye.

Example embodiments of the aforementioned OCT apparatus will now be described in detail with reference to the accompanying drawings.

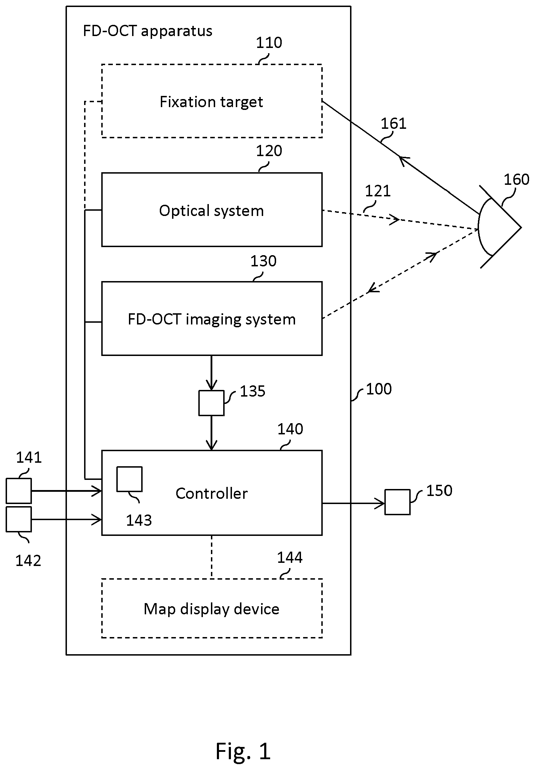

Figure 1 is a schematic illustration of a Fourier-domain OCT (FD-OCT) apparatus 100 arranged to acquire optoretinography (ORG) data 150 that is indicative of a physiological response of a retina of an eye 160 of a subject to an optical stimulus, according to an example embodiment herein. The FD-OCT apparatus 100 comprises a fixation target 110, an optical system 120, an FD-OCT imaging system 130, a controller 140 and a map display device 144. The fixation target 110 and the map display device 144 are optional features that may be omitted in some example embodiments.

The fixation target 110 is arranged to fix a gaze direction 161 of the eye 160 (i.e. a direction away from the eye 160 along the visual axis of the eye 160). This fixation may occur be when the eye 160 fixates on the fixation target 110. That is, the fixation target 110 is arranged to be viewable by the eye 160 so as to fix the gaze direction 161 of the eye 160 when gazed at by the eye 160, during acquisition of the ORG data 150. However, the fixation target 110 may alternatively be arranged to fix the gaze direction 161 of the eye 160 when the other eye of the subject fixates on the fixation target 110. In the majority of subjects that do not have strabismus or the like, the muscles that control eye movement work together and point both eyes in the same direction so that the fixation of the gaze direction of one of the eyes by the fixation target 110 results in the other eye (even when this other eye cannot see the fixation target 110) having the same gaze direction. For example, where the eye 160 is the right eye of the subject then the gaze direction 161 of the eye 160 may fixed by virtue of the left eye of the subject fixating on the fixation target 110. The position of the fixation target 110 relative to the eye 160 may, as in the present example embodiment, be controllable by the controller 140 so as to control a gaze direction 161 of the eye 160 when the eye 160 fixates on the fixation target 110. However, the position of the fixation target 110 may alternatively be set in a fixed position, relative to the expected position of the eye 160 (i.e. the location where the eye 160 is placed relative to the FD-OCT imaging system 130 for the FD-OCT imaging system 130 to acquire the AS-OCT image 130) during manufacture or installation/set-up of the FD-OCT imaging system 130. The fixation target 110 may be integrated into the FD-OCT apparatus 100 so as to be viewable by the eye 160 in various ways that are well-known to those skilled in the art or as is described in US 11,253,146 B2, the contents of which are hereby incorporated by reference in their entirety.

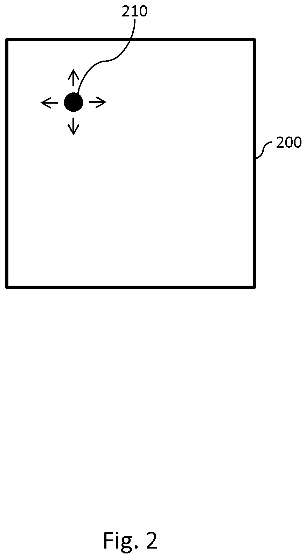

Figure 2 is a schematic illustration of an example implementation of the fixation target 110 in the form of a graphic 210, which is displayed by a display device 200 forming part of the FD-OCT apparatus 100. The display position of the graphic 210 relative to the eye 160 is controllable by the controller 140 so as to control a gaze direction 161 of the eye 160 when the eye 160 fixates on the graphic 210. In other words, the position of the eye 160 relative to the FD-OCT imaging system 130 is fixed (e.g. by placing the patient's chin on a chin rest of the FD-OCT imaging system 130), and the display position of the graphic 210 on the display area of the display device 200 is adjustable, as indicated by the arrows in Figure 2, so as to set (steer) the gaze direction 161 of the eye 160 when the eye 160 fixates on the graphic 210. Although the graphic 210 takes the form of a dot in Figure 2, it will be appreciated that the form of the graphic is not so limited, and may alternatively be provided as a cross, circle or any shape onto which the eye 160 can fixate.

Furthermore, the fixation target 110 may be implemented in a form other than a graphic displayed by a display device 200. For example, the fixation target 110 may include a light source (e.g. a light-emitting diode) attached to an actuator that is controllable by the controller 140 to move the light source relative to the eye 160 so as to control a gaze direction 161 of the eye 160 when the eye 160 fixates on the light source.

The optical system 120 is operable to apply the optical stimulus to the retina of the eye 160 such that the illumination of the retina by the optical stimulus is confined to (and may span or fill) a portion of the retina, the optical system 120 being controllable (optionally while the gaze direction of the eye 160 remains fixed by the fixation target 110) to vary a location on the retina of the eye 160 at which the optical stimulus is to be applied. In other words, the optical system 120 is controllable to apply the optical stimulus to each of a plurality of different portions of the retina of the eye 160 whereby, when the optical stimulus is applied to a portion of the plurality of portions of the retina of the eye 160, the optical stimulus is not applied to any of the other portions of the plurality of portions of the retina. The optical system 120 may be controllable to vary the location on the retina at which the optical stimulus is to be applied while a position of the fixation target 130 relative to the eye 160 remains fixed (and the gaze direction 161 of the eye 160 remains fixed on the fixation target 130), as described in more detail below.

The optical stimulus may, as in the present example embodiment, be a flash of light of a predetermined duration which is applied to the retina of the eye 160 along an optical path 121 of the optical system 120. The duration of the flash of light is typically much shorter than the time period within which OCT data is acquired by FD-OCT imaging system 130 for generating the ORG data 150, and may be dependent on the intensity of the stimulus. The duration of the flash may be between 5 ms and 50 ms, for example. The duration of the optical stimulus may be controlled by the controller 140, as described in more detail below.

The FD-OCT imaging system 130 (which may be a phase-stable FD-OCT imaging system) may, as in the present example embodiment, be a point-scan FD-OCT imaging system. However, the FD-OCT imaging system 130 may take other forms, such as a line-scan or full-field FD-OCT imaging system. The FD-OCT imaging system 130 is operable to acquire complex OCT data 135 by imaging a portion of the retina of the eye 160. The OCT data 135 may, as in the present example embodiment, comprise a temporal sequence of OCT images, such as a temporal sequence of A-scans, B-scans or C-scans, for example.

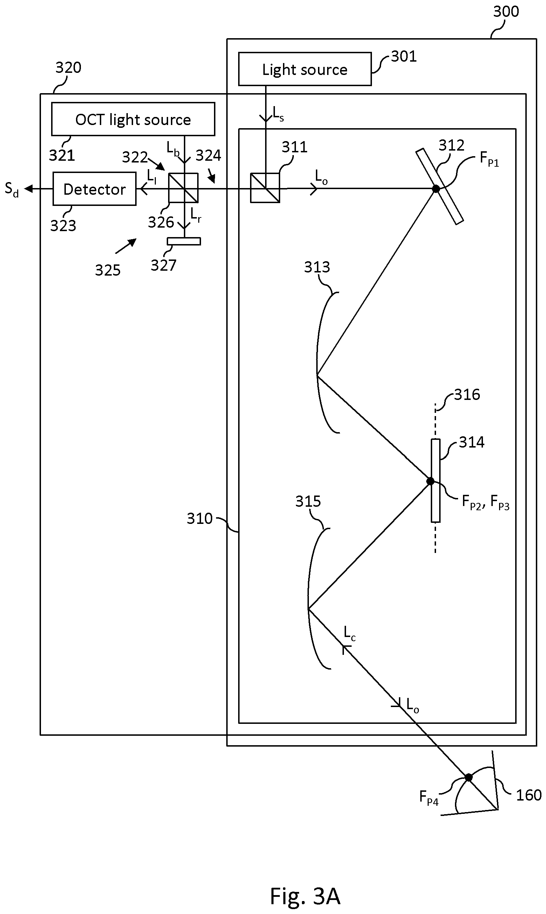

Figure 3A is a schematic illustration of an optical system 300 and an FD-OCT imaging system 320 which may, as in the present example embodiment, form example implementations of the optical system 120 and the FD-OCT imaging system 130 of Figure 1. The optical system 300 comprises a light source 301 and the FD-OCT imaging system 320 comprises an OCT light source 321, an interferometer 322 and a detector 323. A scanning system 310 is shared by both the optical system 300 and the FD-OCT imaging system 320, as described below, and comprises a beam splitter 311, a first scanning element 312, a first curved mirror 313, a second scanning element 314 and a second curved mirror 315. Although a fixation target is not illustrated in Figure 3A for clarity, one may be located in the scanning system 310, on at least one side of the first curved mirror 313, above and/or below the plane of the Figure, as described in US 11,253,146 B2. Light from such a fixation target may thus pass via the second scanning element 314 and then the second curved mirror 315 to the eye 160.

The OCT light source 321 of the FD-OCT imaging system 320 is arranged to generate an OCT beam L_{b}. The OCT light source 321 may, as in the present example embodiment, comprise an illumination source and an illumination source aperture. In this case, the illumination source is arranged to emit light through the light source aperture to generate the OCT beam L_{b}, such that the shape and size (e.g. diameter, in case of the light source aperture being circular) of the illumination source aperture defines the cross-sectional shape and size (e.g. diameter) of the OCT beam L_{b} (i.e. so that these sizes and shapes are the same). The illumination source may, where the FD-OCT imaging system 320 is a swept-source OCT (SS-OCT) imaging system, be a swept illumination source arranged to generate light having a wavelength that is swept over a range of wavelengths during a scan performed by the SS-OCT imaging system or, where the FD-OCT imaging system 320 is a spectral-domain OCT (SD-OCT) imaging system, be a broadband light source which is arranged to generate light simultaneously having a range of wavelengths (i.e. a broad spectral content) during a scan performed by the SD-OCT imaging system. The illumination source may be any known swept or broadband source (as the case may be). For example, the illumination source may comprise a laser or a light emitting diode.

The OCT light source 321 may comprise further components, such as one or more collimating lenses for collimating light from the light source, for example. Further, the OCT light source 321 may alternatively take other forms where the FD-OCT imaging system 320 is a line-field or full-field FD-OCT imaging system as would be readily appreciated by those skilled in the art. For example, where the FD-OCT imaging system 320 is a line-field FD-OCT imaging system, the OCT light source 321 may be arranged to generate a line of light and may comprise a laser and a one or more cylindrical lenses (e.g. combination of a planoconcave lens and a plano-convex lens that are arranged to focus the beam from the laser in respective directions that are orthogonal to one another to form a line of light), although any other kind of spatial light modulator for beam shaping known to those versed in the art may alternatively be employed.

The interferometer 322 is arranged to split the OCT beam L_{b} from the OCT light source 321 to propagate along a sample arm 324 of the interferometer 322, as sample OCT light Lₒ, and to propagate along a reference arm 325 of the interferometer 322, as reference OCT light Lᵣ. The interferometer 322 is further arranged to receive light L_{c} which has been scattered by a portion of the retina of the eye 160 and collected by the scanning system 310, to generate an interference light Lₗ resulting from an interference between the reference OCT light Lᵣ and the collected light L_{c}, and to output the interference light Lₗ to the detector 323. In other words, the reference OCT light Lᵣ propagating along the reference arm 325 and the collected light L_{c} which has been scattered by the portion of the retina of the eye 160 and collected by the scanning system 310 during a scan performed by the FD-OCT imaging system 320 are guided to coincide and interfere with one another, and the resulting interference line of light Lₗ is directed to and received by the detector 323.

The interferometer 322 may, as in the present example embodiment, be a Michelson interferometer using a beam splitter 326 to split the OCT beam L_{b} to propagate along the sample arm 324 and the reference arm 325 of the interferometer 322, and to interfere the reference OCT light Lᵣ that has been reflected from a reference mirror 327 with the collected light L_{c} from the scanning system 310. Although a Michelson interferometer has been described, those skilled in the art will appreciate that the interferometer 322 is not so limited and any interferometer suitable for OCT may be used such as, for example, a Mach-Zehnder interferometer. Further, those skilled in the art will appreciate that the interferometer 322 may also be adapted as appropriate where the FD-OCT imaging system 320 is a line-scan or full-field FD-OCT imaging system. For example, where the FD-OCT imaging system 320 is a line-scan FD-OCT imaging system, the interferometer 322 may be a free-space interferometer using at least one beam splitter. Furthermore, the interferometer 322 is not limited to being a free-space interferometer, and may instead be a fibre-based interferometer. In this case, the interferometer may employ a fibre coupler to split the OCT beam L_{b} to propagate along the sample arm and reference arm of the interferometer 322, and to interfere the reference light L_{R} and the collected light L_{c}.

The scanning system 310 is arranged to perform a (e.g. a one- or two-dimensional) point-scan of the sample OCT light Lₒ across the portion of the retina of the eye 160, and collect light L_{c} which has been scattered by the portion of the retina of the eye 160 during the point scan. The scanning system 310 is therefore arranged to acquire A-scans at respective scan locations that are distributed (e.g. one- or two-dimensionally) across the portion of the retina of the eye 160, by sequentially illuminating the scan locations with the sample OCT light Lₒ, one scan location at a time, and collecting at least some of the light L_{c} scattered by the portion of the retina of the eye 160 at each scan location. By acquiring the successive A-scans, the scanning system 310 is thus able to acquire B-scans and/or C-scans, of the retina of the eye 160.

The sample OCT light Lₒ enters the scanning system 310 from the interferometer 322 and propagates to the beam splitter 311. The sample OCT light Lₒ is then reflected, in sequence, by the first scanning element 312, the first curved mirror 313, the second scanning element 314 and the second curved mirror 315, before being incident on the portion of the retina of the eye 160. The light L_{c} which has been scattered by the portion of the retina of the eye 160 and collected by the scanning system 310 follows the same optical path through the scanning system 310 as the sample OCT light Lₒ, but in reverse order, and exits the scanning system 310 after having propagated via the beam splitter 311.

The point scan is performed by the scanning system 310 by the first scanning element 312 rotating around the first axis (not shown) to scan the sample OCT light Lₒ in a first direction, or a direction opposing the first direction, across the portion of the retina of the eye 160, and/or by the second scanning element 314 rotating around the second axis 316 to scan the sample OCT light Lₒ in a second direction, or in a direction opposing the second direction, across the portion of the retina of the eye 160. The second direction may, as in the present example embodiment, be orthogonal to the first direction. Thus, by rotating the first scanning element 312 and the second scanning element 314, it is possible to steer the sample OCT light Lₒ to any position on the portion of the retina of the eye 160 that is within the field of view of the FD-OCT apparatus 100. As described above, the rotation of the first scanning element 312 and the second scanning element 314 is coordinated by the controller 140, or by a dedicated scanning system controller (not shown), such that the sample OCT light Lₒ is scanned across the portion of the retina of the eye 160 in accordance with a predefined scan pattern. The predefined scan pattern may be any suitable scan pattern known to those versed in the art, for example a unidirectional scan (wherein a set of parallel scan lines are followed in a common direction, along which they extend), a circular scan, a serpentine scan or spiral scan, which may either be present, or at manufacture selected by a user of the FD-OCT apparatus 100.

The first curved mirror 313 and the second curved mirror 315 may, as in the present example embodiment, be respective curved (e.g. ellipsoidal) mirrors each having a first focal point and a conjugate second focal point. The first scanning element 312 is located at the first focal point F_{P1} of the first curved mirror 313, and the second scanning element 314 is located at the second focal point F_{P2} of the first curved mirror 313. The second scanning element 314 is also located at the first focal point F_{P3} of the second curved mirror 315, and the eye 160 is in a vicinity of the second focal point F_{P4} of the second curved mirror 315. More specifically, the pupil of the eye 160 is located at the second focal point F_{P4} of the second curved mirror 315 such that the optical path of the scanning system 310 may be steered in two-dimensions across a region of the retina of the eye 160. However, the first curved mirror 313 and the second curved mirror 315 may be any reflective components having an aspherical reflective surface, such as a shape of a conical section like a parabola or hyperboloid, or may, more generally, have a shape described by one or more polynomial functions of two variables.

The use of curved mirrors in the scanning system 310 allows the FD-OCT imaging system 130 to function as a wide-field FD-OCT imaging system, or an ultra-widefield (UWF) FD-OCT imaging system, as in described in further detail in WO 2014/53824 A1, the content of which is hereby incorporated by reference in its entirety. However, the scanning system 310 is not so limited.

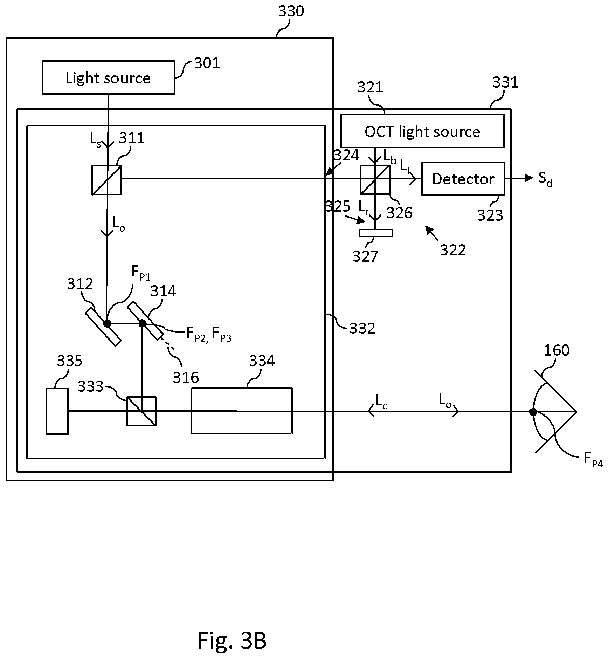

Figure 3B is a schematic illustration of an example optical system 330 and an example FD-OCT imaging system 331 of an alternative implementation, which share a lens-based scanning system 332 (where like reference numerals indicate elements that are the same as in Figure 3A). As shown in Figure 3B, instead of being guided by the curved mirrors 313 and 315, light is directed from the first scanning element 312 to the second scanning element 314 before being directed, via a beam splitter 333, through a lens relay (barrel) 334 comprising one or more lenses to the retina of the eye 160. The beam splitter 333 may take a similar form as the beam splitter 311 (e.g. it may be a beam splitter cube or a dichroic mirror). Further, the scanning system 332 may comprise a first lens relay and a second lens relay (which may leave equal focal lengths) between the first scanning element 312 and the second scanning element 314 (as the only intervening optics). A fixation target 335 may be provided as an example of the fixation target 110 of Figure 1. Light from the fixation target 335 may pass through the beam splitter 333, the lens relay 334 and then to the eye 160.

The first scanning element 312 and the second scanning element 314 may, as in the present example embodiment, each be a galvanometer optical scanner (a "H-galvo" and a "V-galvo", respectively). However, another type of scanning element could alternatively be used, such as a MEMS scanning mirror or a resonant scanning mirror, for example.

The detector 323 is arranged to detect the interference light Lₗ. That is, the detector 323 is arranged to receive the interference light Lₗ from the interferometer 322 and generate a detection signal S_{d} based on the received interference line of light Lₗ. The detector 323 generates the detection signal S_{d} by performing a photoelectric conversion of the interference light Lₗ that is incident on photodetector elements of the detector 323. The specific form of the detector 323 depends on the form in which the FD-OCT imaging system 320 is implemented. For example, where the FD-OCT imaging system 320 is implemented as an SD-OCT imaging system, the detector 322 comprises a spectrometer, which may have a diffraction grating, Fourier transform lend, and a detector array (or a line scan camera). Where the FD-OCT imaging system 320 is implemented as a SS-OCT imaging system, the light detector 120 may comprise a balanced photodetector set-up comprising two photodetectors (e.g. reverse-biased photodiodes), whose output photocurrents are subtracted from one another, with the subtracted current signal being converted into a voltage detection signal by a transimpedance amplifier. The detection signal S_{d} may, as in the present example embodiment, then be processed by OCT data processing hardware of the FD-OCT imaging system 320 to generate the OCT data 135. However, the functions of the OCT data processing hardware may alternatively be performed by the controller 140 (i.e. the detection signal S_{d} may be received and processed by the controller 140 to generate the OCT data 135).

Referring again to Figure 3A, where the FD-OCT imaging system 320 is line-scan system rather than a point scan system, the OCT light source 321 generates a line of light, as described above. In such a case, the interferometer 322 is instead arranged to split the line of light, rather than the OCT beam L_{b}, into the sample OCT light as a sample OCT line of light. The scanning system 310 is arranged to perform a line-scan of the sample OCT line of light across the portion of the retina of the eye 160 and collect light L_{c} which has been scattered by the portion of the retina of the eye 160 during the line scan. Accordingly, the scanning system 310 may further comprise a lens arranged to focus the sample OCT line of light at the first focal point F_{P1} of the first curved mirror 313. The sample OCT line of light is then reflected, in sequence, by the first scanning element 312, the first curved mirror 313, the second scanning element 314 and the second curved mirror 315, before being incident on the portion of the retina of the eye 160, and the light scattered from the portion of the retina of the eye 160 travels back to the detector 323 (i.e. as adapted for a line-scan system) via the scanning system 310, in the same manner described above for the point-scan implementation. The sample OCT line of light may thus be steered, in two dimensions, within the eye by rotating the first scanning element 312 and the second scanning element 314 such that a scan of eye 160 may be performed by the scanning system 310 as coordinated by the controller 140 or the dedicated scanning system controller. However, as a sample OCT line of light is incident on the portion of the retina, the scanning system 310 thus acquires at least one B-scan (e.g. successive B-scans forming a C-scan) of the retina of the eye 160 rather than acquiring at least one A-scan as in the point-scan implementation.

The light source 301 of the optical system 300 is arranged to generate light Lₛ as the optical stimulus. The light Lₛ may, as in the present example embodiment, be of one or more wavelengths in the visible spectrum of the human eye, although it may more generally be of any wavelength(s) for stimulating a physiological response of a retina of an eye 160. The light source 301 may be arranged to generate a plurality of lights of different wavelengths, each of which may be used as the optical stimulus as desired, (e.g. by use of broadband spectrum source, which may produce white light, filtered by at least one tuneable or removable spectral filter) although this may alternatively be achieved by the optical system 300 comprising additional light sources arranged to generate light of different wavelengths to that of the light Lₛ which may be the optical stimulus (these light sources may be combined to a single output of the light source 301 using fibre couplers, wavelength division multiplexing (WDM) fibres, beam splitters or dichroic mirrors).

In addition, the light source 301 may be controllable by the controller 140 to generate the light Lₛ at a desired light intensity.

The light source 301 may, as in the present example embodiment, comprise an illumination source (e.g. a light-emitting diode) and an illumination source aperture in a similar manner to as described above with the OCT light source 321. The light source 301 may comprise further components, such as one or more collimating lenses for collimating light from the light source, for example. The controller 140 controls the light source 301 of the optical system 300 to generate the light Lₛ as the optical stimulus, as described below, and may further control the light source 301 to vary the duration of the optical stimulus provided by the light source 301.

The beam splitter 311 may, as in the present example embodiment, be a cube beam splitter. However, it may instead be a dichroic mirror, for example.

The optical system 300 shares the scanning system 310 used by the FD-OCT imaging system 320 to perform the point-scan. This is achieved by using the beam splitter 311 to couple the optical path along which the light Lₛ travels with the optical path along which the sample OCT light Lₒ travels through the scanning system 310, although any other suitable arrangement for coupling the two optical paths may be used. The optical path along which the light Lₛ travels through the scanning system 310 when generated by the light source 301 may be coupled to the optical path along which the sample OCT light Lₒ travels through the scanning system 310 with the beam splitter 311 such that these optical paths run along a common axis, although the optical paths may alternatively run along axes offset by a predetermined amount (e.g. by adjusting the incident location of one of the optical paths on the beam splitter 311).

The scanning system 310 is thus further arranged to direct the light Lₛ to the retina of the eye 160. The light Lₛ enters the scanning system 110 via the beam splitter 311, and is then reflected, in sequence, by the first scanning element 312, the first curved mirror 313, the second scanning element 314 and the second curved mirror 315, before being applied to the retina of the eye 160.

In the same manner as with the sample OCT light Lₒ, the optical system 300 is controllable to vary a location on the retina of the eye 160 at which the light Lₛ is to be applied by the first scanning element 312 rotating around the first axis (not shown) to move the optical path of the scanning system 310 in the first direction, or in the direction opposite the first direction, across the retina of the eye 160, and by the second scanning element 314 rotating around the second axis 316 to move the optical path of the scanning system 310 in the second direction, or in the direction opposite the second direction, across the retina of the eye 160. By rotating the first scanning element 312 and the second scanning element 314, it is thus possible to steer, in two-dimensions, the optical path along which the light Lₛ travels such that the light Lₛ may be applied to any position on the retina of the eye 160. Accordingly, by controlling the timing and the duration of the light Lₛ generated by the light source 301, and the ranges and rates of rotation of the first scanning element 312 and/or the second scanning element 314, the controller 140 can cause any position on the retina of the eye 160 to be stimulated by the light Iₛ for a required duration of time. This duration of time may be controlled by the controller 140 along with the intensity of the light Lₛ generated by the light source 301 so as to obtain a predetermined degree of bleaching of the retina of the eye 160. For example, the degree of bleaching obtained may be between 10% and 66%. Higher bleaching values may be preferable for studying the alpha wave in the ORG data 150 (i.e. the fast retina response).

By the optical system 300 sharing the scanning system 310 with the FD-OCT imaging system 320, the full field of view of the FD-OCT imaging system 320 may be accessed by the optical system 300 without any additional scanning hardware. This reduces the complexity of the FD-OCT apparatus 100 and may allow easier integration of the optical system 300 into complex sample arms, for example where the FD-OCT imaging system 320 is a UWF FD-OCT imaging system, as described above.

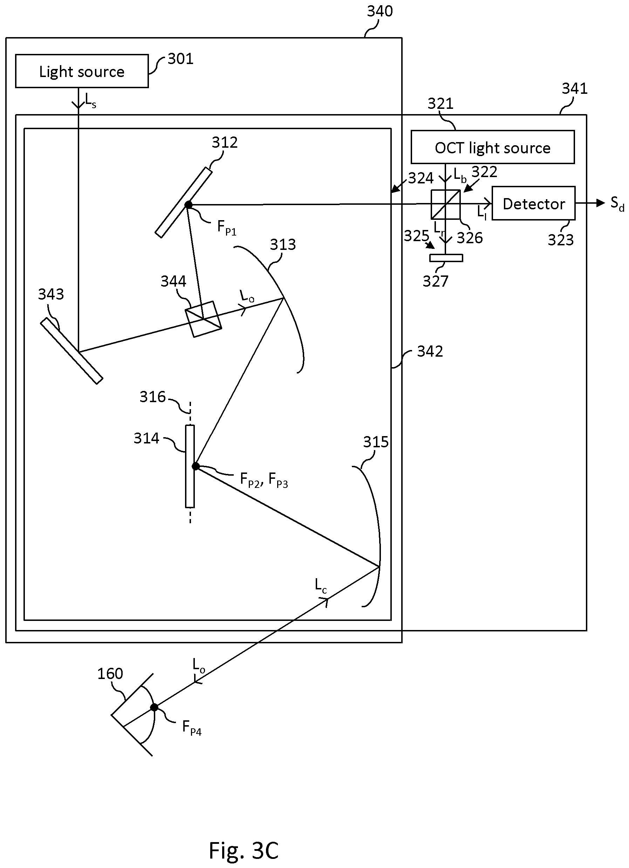

Figure 3C is a schematic illustration of an optical system 340 and an FD-OCT imaging system 341 which may, in accordance with an alternative implementation of the present example embodiment, form example implementations of the optical system 120 and the FD-OCT imaging system 130 in Figure 1 (with like reference numerals indicating the same elements as in Figure 3A). The optical system 340 and the FD-OCT imaging system 341 are the same as the optical system 300 and the FD-OCT imaging system 320 of Figure 3A, respectively, but differ from these components in Figure 3A by sharing the scanning system 342 rather than the scanning system 310. The scanning system 342 is the same as the scanning system 310, other than comprising a third scanning element 343 and a beam splitter 344. The third scanning element 343 may be implemented in the same form as the first scanning element 312 but is used by the scanning system 342 (instead of the first scanning element 312) to direct the light Lₛ from the light source 301 to the retina of the eye 160, and to vary the location on the retina of the eye 160 at which the light Lₛ is to be applied in the first direction and the direction opposing the first direction. The beam splitter 344 may be implemented in the same form as the beam splitter 311, and is located so as to direct the light Lₛ from the light source 301 to the first curved mirror 313, and direct the sample OCT scan Lₒ to the first curved mirror 313 (and the collected light L_{c} to the first scanning element 312). Although a fixation target is not illustrated in Figure 3C for clarity, one may be located in the scanning system 342, on at least one side of the first curved mirror 313, above and/or below the plane of Figure 3C, as described in US 11,253,146 B2. Light from such a fixation target may thus pass via the second scanning element 314 and then the second curved mirror 315 to the eye 160.

As the third scanning element 343 used by the scanning system 342 to direct the light Lₛ to the retina of the eye 160 is different to the first scanning element 312, it may be independently controlled by the controller 140. Accordingly, the optical path along which the light Lₛ travels through the scanning system 342 when generated by the light source 301 can be varied in the first direction (or in the direction opposite the first direction) independently of the optical path along which the sample OCT light Lₒ travels through the scanning system 342. Thus, the optical path along which the light Lₛ travels through the scanning system 342 is coupled to the optical path along which the sample OCT light Lₒ travels through the scanning system 342 in one dimension only, which adds an additional degree of freedom to the variation of the optical stimulus on the retina of the eye 160 as compared to the implementations of Figure 3A and 3B, although at the expense of increasing the complexity of the FD-OCT apparatus 100. For example, this degree of freedom may be advantageous for studying photoreceptor pathways within the retina by removing at least some of the coupling between the optical path of the light Lₛ and the optical path of the sample OCT beam Lₒ (e.g. to introduce a spatial offset between the centre of the first portion and the centre of the second portion of the retina).

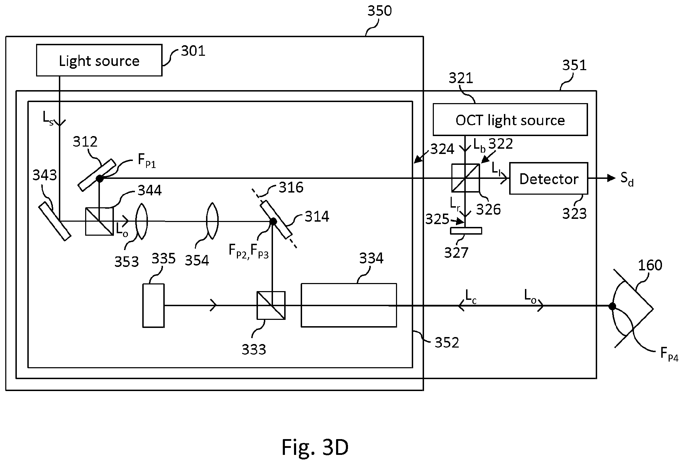

Although the example embodiment of Figure 3C includes the curved mirrors 313 and 315, these may be omitted as shown in Figure 3D, which is a schematic illustration of an example optical system 350 and an example FD-OCT imaging system 351 sharing a scanning system 352 which omits the curved mirrors 313 and 315 (whereby like reference numerals indicate elements that are the same as in Figures 3A to 3C). As shown in Figure 3D, light is directed from the first scanning element 312 and from the third scanning element 343 to the second scanning element 314 via a first lens relay 353 and a second lens relay 354 (which may have equal focal lengths) that are situated between the third scanning element 343 and the second scanning element 314. The first lens relay 353 and the second lens relay 354 are optional, and the only intervening optics between the first scanning element 312 and the second scanning element 314, and between the third scanning element 343 and the second scanning element 314, may be the beam splitter 344 in an alternative example embodiment. As illustrated in Figure 3D, the fixation target 335 may be provided in the scanning system 352 of Figure 3D in a similar way as in the scanning system 332 of Figure 3B.

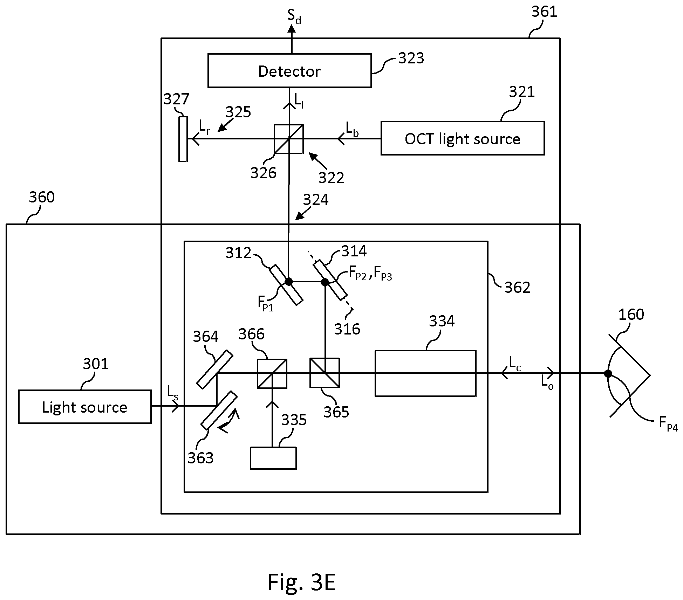

Figure 3E is a schematic illustration of an optical system 360 and an FD-OCT imaging system 361 constituting further alternative implementations of the optical system 120 and the FD-OCT imaging system 130 of the example embodiment. The optical system 360 differs from the optical system 300 of Figure 3A, and the FD-OCT imaging system 361 differs from the FD-OCT imaging system 320 of Figure 3A, only by sharing a scanning system 362 (rather than the scanning system 310). The scanning system 362 is similar to scanning system 332 of Figure 3B, in comprising one or more lenses in a lens relay (barrel) 334 instead of the curved mirrors 313 and 315 of Figure 3A, for example, but differs from scanning system 332 by further comprising a third scanning element 363, a fourth scanning element 364 and a beam splitter 365 instead of beam splitters 331 and 333 of Figure 3B. The third scanning element 363 may be implemented in the same form as the first scanning element 312, and the fourth scanning element 364 may be implemented in the same form as the second scanning element 313, although the third scanning element 363 and the fourth scanning element 364 are used by the scanning system 362 (instead of the first scanning element 312 and the second scanning element 313) to direct the light Lₛ to the retina of the eye 160 and to vary the location on the retina of the eye 160 at which the light Lₛ is to be applied in the first direction, the direction opposing the first direction, the second direction and the direction opposing the second direction. The scanning system 362 may further comprise a first relay lens and a second relay lens (not shown) between the first scanning element 312 and the second scanning element 314, and may comprise a third relay lens and a fourth relay lens (not shown) between the third scanning element 363 and the fourth scanning element 364. The beam splitter 365 may be implemented in the same form as the beam splitter 333, and may be arranged to direct light from the fourth scanning element 364 through the lens barrel 334 towards the eye 160.

The scanning system 362 may further comprise a beam splitter 366, and the fixation target 335 as described above in relation to Figure 3B. Light from the fixation target 335 may be directed by the beam splitter 366 through the beam splitter 365, the lens relay 334 and then to the eye 160.

The third scanning element 363 and the fourth scanning element 364 used by the scanning system 362 to direct the light Lₛ to the retina of the eye 160 are different to the first scanning element 312 and the second scanning element 314 that are used by the scanning system 362 to direct the sample OCT light Lₒ to the retina, and scanning elements 263 and 364 may be controlled by the controller 140 independently of scanning elements 312 and 314. Accordingly, the optical path, along which the light Lₛ travels through the scanning system 362 when generated by the light source 301 can be varied in the first direction (or in the direction opposite the first direction), and in the second direction (or in the direction opposite the second direction), independently of the optical path along which the sample OCT light Lₒ travels through the scanning system 362. Thus, the optical path along which the light Lₛ travels through the scanning system 362 is not coupled to the optical path along which the sample OCT light Lₒ travels through the scanning system 362, allowing the variation of the optical stimulus on the retina of the eye 160to be independently controlled with two degrees of freedom, although at the expense of increasing the complexity of the FD-OCT apparatus 100 as compared to the implementations described above with reference to Figures 3A to 3D.

Although the scanning system 362 comprises a third scanning element 363 and a fourth scanning element 364, these may alternatively be replaced by a single two-dimensional scanner (e.g. a micro-electromechanical system (MEMS) scanner).

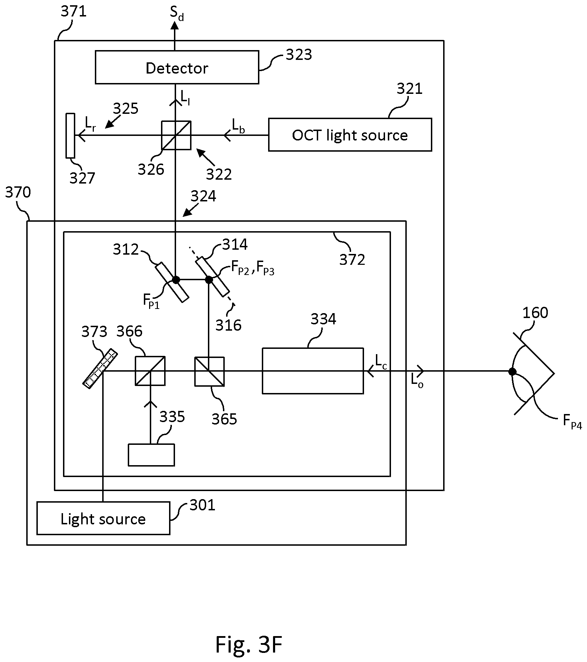

Figure 3F is a schematic illustration of an example optical system 370 and an example FD-OCT imaging system 371 that share a scanning system 372 which comprises a two-dimensional scanner 373. In Figure 3F, elements that are the same as in Figures 3A to 3E are labelled with like numerals. The scanning system 372 is the same as scanning system 362 other than by comprising the two-dimensional scanner 373 (in place of the third scanning element 363 and the fourth scanning element 364), which is used to direct the light Lₛ to the retina of the eye 160 and vary the location on the retina of the eye 160 at which the light Lₛ is to be applied in the first direction, the direction opposing the first direction, the second direction and the direction opposing the second direction.

The scanning system 372 may further comprise a beam splitter 366, and the fixation target 335 as described above in relation to Figure 3B. Light from the fixation target 335 may be directed by the beam splitter 366 through the beam splitter 365, the lens relay 334 and then to the eye 160.

As a further alternative implementation of the example embodiment, the optical system 120 may comprise a spatial light modulator, which is controllable by the controller 140 to vary a location on the retina at which the light L_{S} is to be applied. For example, the spatial light modulator may comprise a projector having the light source 301, a collimator and a dynamic amplitude mask (e.g. in the form of a digital micromirror device (DMD), which is arranged to be illuminated by the collimated light and is controllable by the controller 140 to allow the collimated light L_{S} generated by the light source 301 to pass via only a predefined portion of the dynamic amplitude mask so as to vary the location on the retina where light from the light source 301 is incident.

Where the dynamic amplitude mask is provided in the form of a DMD, the DMD may comprise an array of rotatable micromirrors, which are individually controllable by the controller 140 to switch from being in one of a first and a second, different orientation to the other of the first and second orientation. More specifically, the DMD may be configured to set each micromirror in the DMD either to a first orientation, to reflect light from the light source 301 towards the eye 160, or to a second orientation such as to reflect incident light away from the eye 160 and thus prevent light from the light source 301 from reaching the eye 160. In this manner, the use of DMD allows binary amplitude modulation of the light received at each micromirror position on the DMD.

It should be noted, however, that the functionality of the dynamic amplitude mask may be provided by any suitable type of spatial light modulator other than a DMD, such an array of liquid crystal cells, or an analog micromirror array, for example. For example, in an alternative example embodiment, where the dynamic amplitude mask comprises an array of liquid crystal cells, the liquid crystal in each liquid crystal cell of the array may be individually switchable between a first liquid crystal phase and a second liquid crystal phase. A liquid crystal cell that is in the first liquid crystal phase transmits light L_{S} incident thereon towards the eye 160. A liquid crystal cell that is in the second liquid crystal phase, on the other hand, blocks incident light L_{S}, preventing it from being transmitted to the eye 160. Furthermore, the unmasked portion of the dynamic amplitude mask may consist of liquid crystal cells of the array having liquid crystals in the first phase, while the masked portion of the dynamic amplitude mask may comprise liquid crystal cells of the array having liquid crystals in the second phase. The spatial light modulator may, as a further example, comprise an array of light sources (e.g. LEDs) that are arranged to provide corresponding collimated, spatially separated beams of light, and which are controllable by the controller 140 so as to provide control of the location(s) on the retina at which the optical stimulus is applied.

In addition, the spatial light modulator may be used to vary the location on the retina of the eye 160 at which the light L_{S} is to be applied when the FD-OCT imaging system 130 is a full-field FD-OCT imaging system, by way of example.

It should be noted that, although the above-described arrangements for varying the location on the retina at which the optical stimulus is to be applied are described within the context of an FD-OCT imaging system 130 which is arranged to deliver a single sample OCT beam Lₒ to the retina of the eye 160, the present disclosure is not so limited, and these arrangements for varying the location on the retina at which the optical stimulus is to be applied may also be used within multi-beam FD-OCT imaging systems that are arranged to simultaneously deliver multiple sample OCT beams to the retina.

The above-described arrangements may allow the location on the retina at which the optical stimulus is applied to be set with greater accuracy than in a case where this location is set by eye steering, e.g. by the fixation target 110 being moved laterally relative to the eye 160 (i.e. in the field of view of the eye 160) to vary the location on the retina at which the optical stimulus is applied. In addition, keeping the fixation target 110 at a fixed location during the acquisition of the ORG data 150 may improve patient comfort and improve the ease of use of the system. Further, some of the above-described arrangements for varying the location on the retina at which the optical stimulus is to be applied make use of the existing optics the OCT sample arm, thus enabling easier integration of the optical system 120 into existing OCT systems.

Returning to Figure 1, the controller 140 is arranged to acquire a first indicator 141 which is indicative of a duration of the physiological response of the retina in the ORG data 150 that is to be acquired by the FD-OCT apparatus 100. In other words, the first indicator 141 indicates how long the response of the retina (e.g. in terms of a change in optical path length of the outer segment (OS) or other retinal layer) to a light stimulus is to be monitored for to generate the ORG data 150. The controller 140 may, as in the present example embodiment, be arranged to acquire the first indicator 141 by selecting a value from a group of values comprising (or in some cases consisting of) a first value, which is indicative of a duration less than 20 ms of the physiological response of the retina in the ORG data 150 that is to be acquired by the FD-OCT apparatus 100, and a second value, which is indicative of a duration greater than 20 ms (e.g. between 20 ms and 300 ms) of the physiological response of the retina in the ORG data 150 that is to be acquired by the FD-OCT apparatus 100.

The first value may be appropriate for measuring the fast component of the response associated with the initial contraction and subsequent elongation of the OS that occur on a timescale of a few milliseconds to a few tens of milliseconds, while the second value may be appropriate for measuring the following component of the response which is associated with the further, slower elongation of the OS and occurs on a timescale of a few seconds.

The controller 140 may acquire the first indicator 141 by the user of the FD-OCT apparatus 100 selecting a value from the group of values mentioned above. For example, the indicator 141 may be input to the controller 140 by the user selecting one of the aforementioned values by selecting the corresponding graphical representation of the value from a group of graphical representations that represent the group of values (e.g. from a group of labels including "Fast response" and "Slow response") being displayed on the map display device 144 (or an external display, such as a computer screen or the like), using a mouse and/or keyboard, or via one or more touch interactions in case the display is a touchscreen, for example. As an alternative, the controller 140 may acquire the first indicator 141 by automatically selecting a value (e.g. the first value) from the group of values mentioned above, depending on a type of disease or disorder (e.g. retinitis pigmentosa, central serous retinopathy, Stargardt's disease or age-related macular degeneration (AMD), which have been linked to a reduction in amplitude and/or faster completion of the initial contractile phase) indicated on the map display device 144 or other display and selected by the user using an input means of the kinds mentioned above, for example.

The controller 140 is further arranged to acquire a number N of second indicators 142, each second indicator being indicative of a respective target location on the retina at which the optical stimulus is to be applied by the optical system 120. Here, N is an integer greater than or equal to 2.

The inventors have recognised that the tendency of the pupil of the eye 160 to contract in response to applied optical stimuli and involuntary movements of the subject that inevitably occur lead to a growing risk of the optical stimuli from the optical system 120 and/or the OCT light being clipped by the pupil as the acquisition of ORG data 150 progresses. The inventors have further recognised that these factors cause the desired measurement timescale for the retinal response to limit the amount of OCT data 135 that can be acquired by the FD-OCT imaging system 130 before the quality of the OCT data 135 and consequently the ORG data 150 becomes degraded by the clipping. To reduce the risk of such degradation of the acquired ORG data 150, the inventors have recognised that the number (N) of target locations indicated by the acquired second indicators 142 needs to vary in dependence on the first indicator 141, such that N decreases as the duration (or timescale) indicated by the first indicator 141 increases. Thus, the longer the desired measurement timescale for the retinal response, the fewer locations on the retina should be targeted for OCT measurements.

The controller 140 is preferably arranged to vary the number N of second indicators 142 that it acquires, based on the acquired first indicator 141, such that, within the time window imposed by the factors noted above, for example, the controller 140 uses the N second indicators 142 to control the optical system 120 to apply the optical stimulus to the respective first portions of the retina, and controls the FD-OCT imaging system 130 to acquire the respective OCT data 135 for each of the first portions of the retina over the duration (or timescale) indicated by the first indicator 141. In this case, all of the OCT data 135 can be acquired from the N second portions of the retina within a time interval which is small enough to avoid or minimise the clipping of the optical stimulus and/or the OCT light by the pupil of the eye 160. In such example embodiments, the controller 140 may store in a storage device a third indicator 143 which is indicative of a period of time over which the optical system 120 is to apply the optical stimulus to respective first portions of the retina and the FD-OCT imaging system 130 is to acquire, for each of the first portions of the retina, the respective OCT data 135 of the respective second portion of the retina over the duration indicated by the first indicator 141. Furthermore, in such example embodiments, the controller 140 may be arranged to determine, based on the first indicator 141 and the third indicator 143, the number, N, of second indicators 142 to be acquired such that, within the period of time indicated by the third indicator 143, the controller 140 uses the N second indicators 142 to control the optical system 120 to apply the optical stimulus to the respective first portions of the retina, and the controller 140 controls the FD-OCT imaging system 130 to acquire the respective OCT data 135 for each of the first portions of the retina.

The controller 140 may be arranged to update the third indicator 143 based on an estimated or measured size of the pupil of the eye 160 such that the period of time indicated by the third indicator 143 increases as the size of the pupil increases. The size of the pupil may be expressed in any suitable or desirable form (for example, in terms of the diameter, radius or area of the pupil), and may be entered by the user using any appropriate user interface device (e.g. keyboard, mouse, touchscreen, etc.) or determined automatically by the controller 140 processing one or more images of the pupil, which have been acquired by one or more cameras forming part of the FD-OCT apparatus 100 (e.g. one or both cameras of a stereo camera system that may be employed to measure the distance (along the axial (z) direction) of the pupil from an exit pupil of the FD-OCT apparatus 100), using measurement techniques well-known to those versed in the art. The period of time indicated by the third indicator 143 preferably does not exceed 300 ms.

The controller 140 is arranged to use the second indicators 142 to control the optical system 120, preferably while a position of the fixation target 110 relative to the eye 160 remains fixed, to apply the optical stimulus to respective first portions of the retina at the respective target locations. The controller 140 is then arranged to control the FD-OCT imaging system 130 to acquire, for each of the first portions of the retina, respective OCT data 135 of a respective second portion of N second portions of the retina over the duration indicated by the first indicator 141, wherein at least a part of the respective second portion of the retina is disposed in relation to the respective first portion of the retina so as to be stimulated by the applied optical stimulus during acquisition of at least some of the respective OCT data 135. The controller 140 is further arranged to process the respective OCT data 135 of each second portion of the retina to generate respective ORG data 150 indicative of a respective physiological response of the second portion of the retina to the optical stimulus applied to the corresponding first portion of the retina, as described in more detail below.

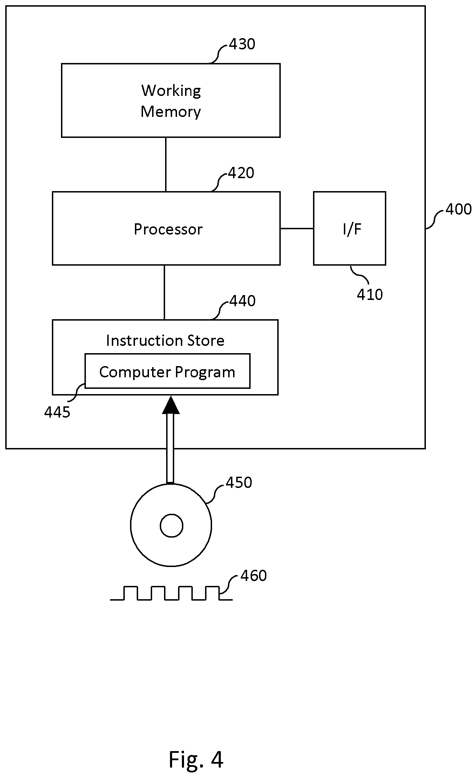

The controller 140 (and the dedicated scanning system controller, where provided) may be provided in any suitable form, for example as a programmable signal processing hardware 400 of the kind illustrated schematically in Figure 4. The programmable signal processing apparatus 400 comprises a communication interface (I/F) 410, for receiving the OCT data 135 (or the detection signal S_{d}) from the FD-OCT imaging system 130 and, in some cases, receiving the first indicator 141 and the second indicators 142, outputting control signals to the FD-OCT imaging system 130 and outputting the ORG data 150 and/or a graphical representation thereof to the map display device 144 (such as a computer screen or the like) to be displayed thereby. The signal processing hardware 400 further comprises a processor 420 (e.g. a Central Processing Unit, CPU, and/or a Graphics Processing Unit, GPU), a working memory 430 (e.g. a random-access memory) and an instruction store 440 storing a computer program 445 comprising the computer-readable instructions which, when executed by the processor420, cause the processor 420 to perform various functions including those of the controller 140 described herein. The working memory 430 stores information used by the processor 420 during execution of the computer program 445. The instruction store 440 may comprise a ROM (e.g. in the form of an electrically erasable programmable read-only memory (EEPROM) or flash memory) which is pre-loaded with the computer-readable instructions. Alternatively, the instruction store 440 may comprise a RAM or similar type of memory, and the computer-readable instructions of the computer program 445 can be input thereto from a computer program product, such as a non-transitory, computer-readable storage medium 450 in the form of a CD-ROM, DVDROM, etc. or a computer-readable signal 460 carrying the computer-readable instructions. In any case, the computer program 445, when executed by the processor 420, causes the processor 420 to perform the functions of the controller 140 as described herein. In other words, the controller 140 of the example embodiment may comprise a computer processor 420 and a memory 440 storing computer-readable instructions which, when executed by the computer processor 420, cause the computer processor 420 to acquire the first indicator 141 and the N second indicators 142 described above, to use the N second indicators 142 to control the optical system 120 (preferably while the position of the fixation target 110 relative to the eye 160 remains fixed) to apply the optical stimulus to respective first portions of the retina at the respective target locations, to control the FD-OCT imaging system 130 to acquire, for each of the first portions of the retina, respective OCT data 135 of a respective second portion of N second portions of the retina over the duration indicated by the first indicator 141, and to process the respective OCT data 135 of each second portion of the retina to generate the respective ORG data 150 indicative of a respective physiological response of the second portion of the retina to the optical stimulus applied to the corresponding first portion of the retina, as described herein.

It should be noted, however, that the controller 140 may alternatively be implemented in non-programmable hardware, such as an ASIC, an FPGA or other integrated circuit dedicated to performing the functions of the controller 140 described above, or a combination of such non-programmable hardware and programmable hardware as described above with reference to Figure 4. Further, while the controller 140 is described in relation to a single programmable signal processing hardware 400, the controller 140 is not so limited and its functions may be split between a plurality of programmable signal processing hardware of the kind shown in Figure 4, or parts thereof, and/or the aforementioned non-programmable hardware. For example, the controller 140 may be implemented by programmable signal processing hardware of the kind shown in Figure 4 which is arranged to acquire the first indicator 141 and the N second indicators 142 described above, to use the N second indicators 142 to control the optical system 120 (preferably while the position of the fixation target 110 relative to the eye 160 remains fixed) to apply the optical stimulus to respective first portions of the retina at the respective target locations, to control the FD-OCT imaging system 130 to acquire, for each of the first portions of the retina, respective OCT data 135 of a respective second portion of N second portions of the retina over the duration indicated by the first indicator 141, as described herein. In this case, a separate processor (e.g. one similar to processor 420 in Figure 4) may be arranged to receive the acquired OCT data 135 from the controller 140 and generate the ORG data 150 based on the acquired OCT data 135 (and output the ORG data 150 to, for example, the map display device 144), as described herein.

The map display device 144 may, as in the present example embodiment, be arranged to receive data from the controller 140 for displaying to the user (e.g. a clinician), for example, and the communication interface 410 may be arranged to receive inputs from the user of the FD-OCT apparatus 100 such as those as required by the controller 140. For example, the map display device 144 may receive and display the ORG data 150, or a graphical representation thereof, or may be controlled by the controller 140 to display the maps described herein below. The inputs from the user of the FD-OCT apparatus 100 may include, for example, the first indicator 141 and optionally also the second indicators 142, which the map display device 144 may transmit to the controller 140. The map display device 144 may be an LCD screen, for example, and may comprise programmable signal processing hardware 400 of the kind illustrated schematically in Figure 4, which may be used to generate the graphical representation of the ORG data 150, process inputs provided by the user (e.g. by processing touch inputs from the user where the map display device 144 is a touchscreen) and interface with the controller 140. However, the map display device 144 is optional and may be omitted. For example, the aforementioned functions of the map display device 144 may be provided by an external computer or, in some cases, there may be no need to display the ORG data 150 and it may instead be stored on an external server or within the memory of the controller 140 for later retrieval.

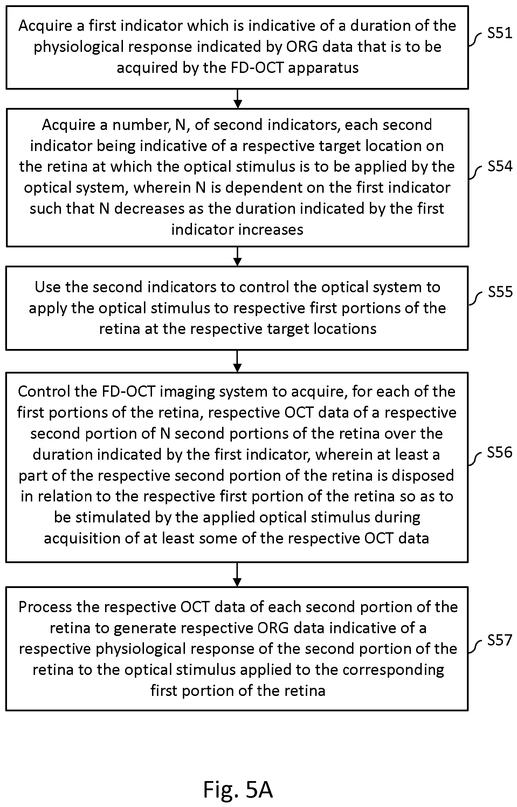

Figure 5A is a flow diagram illustrating a process by which the controller 140 may, as in the present example embodiment, generate the ORG data 150. This process defines an ORG capture session, which may (but need not) be initiated by a period of dark adaptation of the retina of the eye 160. For example, the FD-OCT apparatus 100 may be arranged to prevent light (both internal to and external to the FD-OCT apparatus 100) from being incident on the retina of the eye 160 for a predetermined duration of time (e.g. approximately 5 minutes) or the patient may, more simply, be instructed to close their eyes or wait in a dark room for the predetermined duration of time.

In process S51 of Figure 5A, the controller 140 acquires a first indicator 141 which is indicative of a duration of the physiological response in ORG data that is to be acquired by the FD-OCT apparatus 100, for example as discussed above.

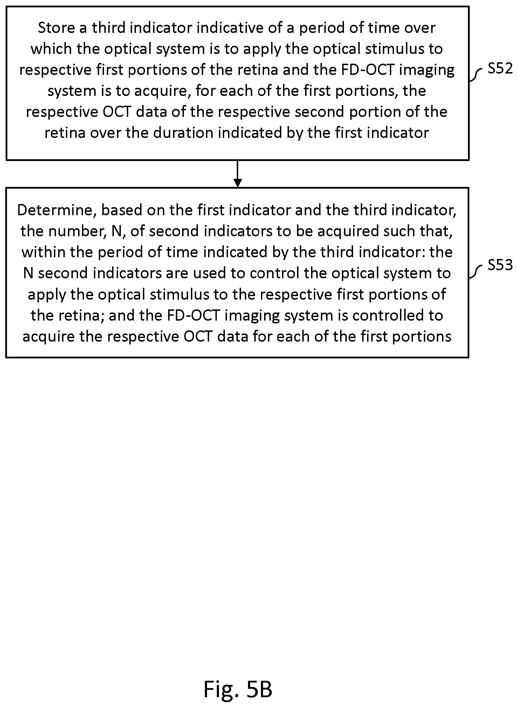

In optional process S52 shown in Figure 5B, the controller 140 stores a third indicator 143, which is indicative of a period of time over which the optical system 120 is to apply the optical stimulus to respective first portions of the retina and the FD-OCT imaging system 130 is to acquire, for each of the first portions of the retina, the respective OCT data 135 of the respective second portion of the retina over the duration indicated by the first indicator 141.

In optional process S55 shown in Figure 5B, the controller 140 determines, based on the first indicator 141 and the third indicator 143, the number, N, of second indicators 142 to be acquired such that, within the period of time indicated by the third indicator 143, the N second indicators 142 are used to control the optical system 120 to apply the optical stimulus to the respective first portions of the retina, and the FD-OCT imaging system 130 is controlled to acquire the respective OCT data 135 for each of the first portions of the retina. The controller 140 may, for example, determine N so as to maximise the number of first portions and corresponding second portions that are stimulated by the optical system 120 and targeted for measurement by the FD-OCT imaging system 130, respectively, within the period of time indicated by the third indicator 143.

Referring again to Figure 5A, in process S54, the controller 140 acquires a number N of second indicators 142, each second indicator being indicative of a respective target location on the retina of the eye 160 at which the optical stimulus is to be applied by the optical system 120, where N is an integer greater than or equal to 2 and is dependent on the first indicator 141 as described above. For example, each of the N second indicators 142 may be indicative of a respective location of a point on the retina of the eye 160 at which the optical stimulus applied by the optical system 120 is to be centred on. The second indicators 142 may be generated automatically by the controller 140, in accordance with a predetermined scan pattern for the optical system 120 that traverses the target locations, the scan pattern being stored (e.g. in working memory 430 or instruction store 440) in association with each of a plurality of values of the first indicator 141 (or of possible values of N, where optional process S53 of Figure 5B is performed). The second indicators 142 may additionally be generated relative to a predetermined location on the retina defined in relation to one or more anatomical landmarks (e.g. the optic disk, macula, etc.). The second indicators 142 may alternatively be input to the controller 140 by the user selecting the target locations on an image of the retina (e.g. an en-face OCT image previously acquired by the FD-OCT imaging system 130 or a reflectance image of the retina previously acquired using a scanning laser ophthalmoscope (SLO), which may be included in the FD-OCT imaging apparatus 100 and share the scanning system 310 with the FD-OCT imaging system 130, for example) displayed on the map display device 144 (or an external display, such as a computer screen or the like), using a mouse and/or keyboard, or via one or more touch interactions in case the display is a touchscreen, for example.

In process S55 of Figure 5A, the controller 140 uses the acquired second indicators 142 to control the optical system 120, preferably while a position of the fixation target 110 relative to the eye 160 remains fixed and the eye 160 fixates on the fixation target 110 (i.e. while a location of the fixation target in a field of view (FoV) of the eye 160 remains fixed, where the FoV covers everything the eye 160 is able to see as it pivots in its eye socket while the head remains in a fixed orientation), to apply the optical stimulus to respective separate first portions of the retina of the eye 160 that are at the respective target locations. The controller 140 may be configured to transform each location on the retina indicated by the respective second indicator 142 into a corresponding set of one or more control parameters for steering the optical system 120 to apply the optical stimulus at substantially the same location on the retina as that indicated by the second indicator 142. This can be done in one of several different ways. For example, the controller 140 may use a mapping between the locations on the retina and corresponding values of the control parameters, which may be provided in the form of a look-up table (LUT) or a function defined by a set of parameters, for example. The mapping may be determined by calibration, using techniques known to those skilled in the art. The optical stimulus applied to each of the first portions of the retina provides no stimulation in any of the other first portions of the retina of the eye 160. That is, each of the second indicators 142 is of a respective target location on the retina which is different to the target locations indicated by the other second indicators 142, so that each of the first portions of the retina do not overlap on the retina with any of the other first portions. Each of the first portions of the retina is smaller than the field of view of the FD-OCT imaging system 130 (e.g. less than 0.1, 1 or 10% of the area covered by the field of view of the FD-OCT imaging system 130). Examples of the first portions of the retina are described in more detail below.

In the present example embodiment, as shown in Figure 3A, the controller 140 controls the scanning system 310 to rotate the first scanning element 312 and the second scanning element 314 such that an optical path, along which the light Lₛ travels through the scanning system 310, is incident upon each of the target locations on the retina, and the controller 140 controls the light source 301 to generate the light Lₛ so as to propagate along the optical path either after the aforementioned rotation or, as the case may be, during the aforementioned rotation.

In process S56 of Figure 5A, the controller 140 controls the FD-OCT imaging system 130 to acquire, for each of the first portions of the retina, respective OCT data 135 of a respective second portion of the N second portions of the retina over the duration indicated by the first indicator 141. The controller 140 may acquire each of the second indicators 142 by the user selecting the location of the second portion of the retina on the map display device 144 as described above. Alternatively, the second indicator 142 may be generated by the controller 140 based on at least one of the first indicator 141 or the first portion of the retina of the eye 160, as described herein. At least a part of each second portion of the retina is disposed in relation to its corresponding first portion of the retina so as to be stimulated by the optical stimulus applied to the first portion during acquisition of at least some of the respective OCT data. Each second portion may have a pre-determined, fixed positional relationship to the corresponding first portion, for example. Each second portion of the retina may be separate (spaced apart) from every other second portion of the retina. However, at least some of the second portions may, in some cases, at least partially overlap which at least some of the other second portions.

For each first portion, the acquisition of the OCT data 135 from the corresponding second portion may, as in the present example embodiment, be of a temporal sequence of OCT images of the second portion of the retina within a first period of time (i.e. a period of time between the time of acquisition of the first OCT image in the temporal sequence of OCT images and the time of acquisition of the last OCT image in the temporal sequence of OCT images) that is indicated by the first indicator 141. Accordingly, in the present example embodiment as shown in Figure 3A, the controller 140 controls the rotation of one or both of the first scanning element 312 and the second scanning element 314 to perform a point scan of the second portion of the retina of the eye 160 in accordance with the predetermined scan pattern to acquire the temporal sequence of OCT images. The controller 140 may alternatively instruct the dedicated scanning system controller (where provided) to control one or both of scanning elements 313 and 314.

As noted above, at least a part of each second portion of the retina of the eye 160 is disposed in relation to the corresponding first portion so as to be stimulated (or at least partially illuminated) by the applied optical stimulus during acquisition of at least some of the OCT data 135 from the second portion of the retina. That is, for the performance of process S56 for each second portion of the retina, the controller 140 previously performs process S54 and uses the acquired second indicator 142 to control the optical system 120 in process S55 to apply the optical stimulus to the corresponding first portion of the retina of the eye 160 at a first time which falls within the first period of time so that at least some of the OCT images in the temporal sequence of OCT images are acquired after the first time, and at least a part of the second portion of the retina of the eye 160 in each of these OCT images is stimulated (or at least partially illuminated) by the applied optical stimulus. The second portion of the retina may at least partially overlap the corresponding first portion of the retina, or it may alternatively be disposed away from but sufficiently close to the corresponding first portion for the light incident on the first portion to scatter into at least some of the second portion or otherwise cause at least a part of the second portion of the retina to be provided with an optical stimulus for stimulating the region of the retina therein. Examples of the location of second portions of the retina of the eye 160 relative to corresponding first portions of the retina of the eye 160 are described in more detail below. In a variant of the example embodiment, the controller 140 may first acquire an indication of the location of one or more of the second portions of the retina, as described above, at which the OCT data 135 is to be acquired, and may subsequently generate the corresponding second indicator(s) 142 such that the first portion(s) of the retina, which is/are at the target location(s), stimulate at least a part of the second portion(s) of the retina (i.e. such that the ORG data 150 may be generated at the location of the second portion of the retina). For example, the location of a first portion may be selected to lie along a predetermined scan pattern which is used to acquire the OCT data 135. Accordingly, the same rotation of one or both the first scanning element 312 and the second scanning element 314 in Figure 3A or 3B, or the second scanning element 314 in Figure 3C or 3D, for example, may be used to both acquire the OCT data 135 and apply the optical stimulus to the first portion of the retina. This may allow the optical stimulus to be applied to the eye 160 within a smaller time window than may be possible where the controller 140 needs to coordinate differing rotations of the scanning elements in process S54 and S55, which may be important when the temporal sequence of OCT images acquired in process S56 are closely spaced in time (as may be desired to, for example, reduce motion artefacts in the OCT images). Note that this advantage can similarly be achieved where the location of the first portion does not lie along the predetermined scan pattern on the retina but is displaced from the scan pattern by use of an arrangement as described above with reference to Figures 3C to 3F (although at the expense of increasing the system complexity).

Referring again to Figure 5A, in process S57, the controller 140 processes the respective OCT data 135 of each second portion of the retina to generate respective ORG data 150 indicative of a respective physiological response of the second portion of the retina to the optical stimulus applied to the corresponding first portion of the retina. The controller 140 may, as in the present example embodiment, generate the ORG data 150 based on phase information in the acquired OCT data 135. The ORG data 150 generated by the controller 140 may be displayed on the map display device 144 to the user or output to an external computer or server for analysis, for example.

The controller 140 may generate the ORG data 150 based on the acquired OCT data 135 using any of the techniques known to those skilled in the art, for example, the velocity-based ORG technique described in Kari V. Vienola, et al., "Velocity-based optoretinography for clinical applications," Optica 9, 1100-1108 (2022), the content of which is herein incorporated by reference in its entirety. In brief, this velocity-based ORG technique generates ORG data based on acquired OCT data (which, in this case, comprises a temporal sequence of B-scans) by firstly flattening each of the B-scans such that the photoreceptor inner and outer segment (IS/OS) and cone outer segment (COST) reflections lie at the same height for each A-scan in each respective B-scans. Then, a moving (e.g. 10 ms) time window is used to select a group of (e.g. five) sequential B-scans with motion corrected relative to the first B-scan in the series. The phase data cube of each complex data cube for each spatial coordinate pair in the volume is then unwrapped in the temporal dimension to minimise the magnitude of the difference in phase between data cubes of consecutive phase B-scans. After unwrapping, a rate of phase change is calculated for each coordinate pair by using a least-squares linear fit with respect to time to calculate the instantaneous velocity for each spatial location. These instantaneous velocities and the B-scan amplitude, if desired, are averaged in the lateral dimension to give instantaneous, depth-dependent measures of velocity and backscattering, respectively. By shifting the (10 ms) time window a time series of depth profiles is constructed, separately for velocity and reflectively. Both of these may be visualised in time-depth coordinates, as M-scans. The velocities of the IS/OS and COST layers are subsequently extracted and the difference between them is the rate of the contraction/elongation of the OS in the region as a function of time, which may form the ORG data 150 (or, alternatively, the OS length response may be the ORG data 150). However, these techniques may be applied to other retinal layers such as, for example, the rod photoreceptors by extracting the velocities of the IS/OS and POST layers. Alternatively, the magnitude or another characteristic of the so-called "alpha wave" (i.e. the fast retinal response, which is typically on a timescale of a few milliseconds) in the data of the rate of contraction/elongation of the outer segment (OS) as a function of time may be determined and saved as (or as a part of) the ORG data 150. Additionally or alternatively, the magnitude or another characteristic of the so-called "beta wave" (i.e. the slow response, which is typically on a timescale of a few seconds) may be determined and saved as (or as a part of) the ORG data 150. As a further alternative, the ORG data 150 of each second portion of the retina may comprise a value (e.g. on a predefined scale, for example a scale of 1 to 10) which is indicative of a quality of a retinal response at the location of the second portion on the retina which is generated by comparing the retinal response indicated by the acquired ORG data 150 with the response of a healthy retina. The healthy retinal response may be obtained from ORG data 150 at a location on the retina of the eye 160 which is assessed by a clinician to be healthy or may be obtained from ORG data of a sample healthy eye, for example.

The controller 140 may generate the ORG data 150 based on the acquired OCT data 135 using intensity-based ORG techniques such as, for example, the OCT brightness change and OCT band analysis techniques described in Kim T-H, Ma G, Son T and Yao X, "Functional Optical Coherence Tomography for Intrinsic Signal Optoretinography: Recent Developments and Deployment Challenges", Front. Med. 9:864824, (2022), the contents of which are incorporated by reference herein in their entirety. OCT brightness change techniques may be used to detect local variations in pixel intensity value caused by the light stimulus on the retina. The data processing technique used in OCT brightness change analysis techniques may comprise registering raw OCT B-scans to account for eye movements, normalizing the pixel intensities based on the inner retinal intensity to limit the effect of pupillary response, identifying "active" intrinsic optical signal (IOS) pixels (i.e. pixels exhibiting a significant change in intensity after the light stimulus, which can be positive where intensity increased or negative where intensity decreased) and quantifying the number of these active IOS pixels for analysis. OCT band analysis techniques may include, for example, deconvolution methods for band analysis (e.g. of the hyper- and hypo-reflective bands in the retina). Where intensity-based ORG techniques are used, the OCT data may be acquired by OCT imaging systems which are not phase stable.

It is noted that the controller 140 may, as in the present example embodiment, initially acquire a first of the N second indicators 142 and use this second indicator to acquire first OCT data corresponding to the indicator. The controller 140 may then repeat this OCT data acquisition process (workflow) using each of the remaining N-1 second indicators until respective OCT data has been acquired for each of the remaining second indicators 142. The controller 140 may then process the respective OCT data 135 to generate the respective ORG data 150 in process S57 of Figure 5A. However, the generation of the ORG data 150 may be performed in parallel with the acquisition of the respective OCT data 135. That is, after the first respective OCT data is acquired, the controller 140 may begin performing process S57 to generate respective ORG data 150 based on the first respective OCT data whilst repeating the aforementioned workflow for each of the remaining second indicators 142, generating respective ORG data 150 based on the respective OCT data 135 corresponding to each of the second indicators in parallel as it is acquired. Further, process S54 of Figure 5A may be performed to acquire all of the second indicators 142 before any of processes S55, S56 and S57 is performed for the first of the N second indicators 142.

The controller 140 may perform processes S55 to S57 of Figure 5A (for all the second indicators 142) during the ORG capture session after only a single period of dark adaptation of the retina of the eye 160. This is made possible by a combination of the applied optical stimulus being confined to a first portion of the retina which is smaller than the field of view of the FD-OCT imaging system 130, and the location of the applied optical stimulus on the retina being changeable among a plurality of different locations on the retina by the optical system 120. As the optical stimulus applied to each of the first portions of the retina provides no stimulation in any of the other first portions of the retina, the need to provide a period of dark adaptation after each application of the optical stimulus to the retina (as with the existing ORG techniques described in the background section) is avoided. Further, the controller 140 may perform processes S55 to S57 (for all of the second indicators) to acquire ORG data for a plurality of retinal locations on a much smaller timescale than that required for light adaptation, (e.g. within a period not exceeding 0.1, 1 or 3 seconds, for example), which is not achievable using the conventional approaches described above.

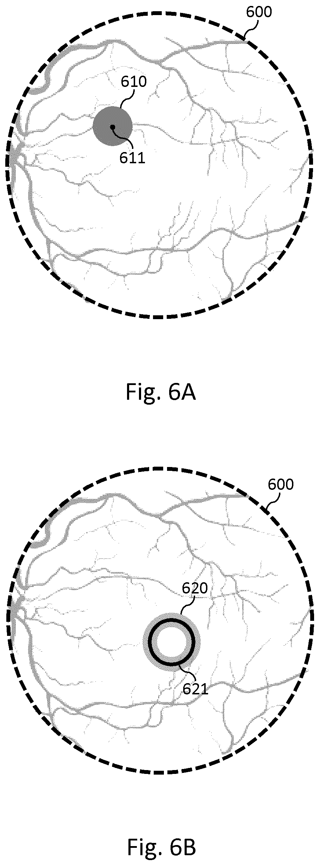

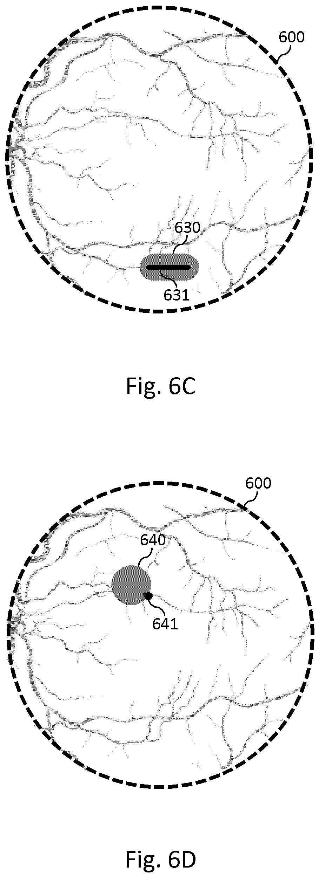

Figures 6A, 6B and 6C are schematic illustrations of example first portions of the retina of the eye 160 and example corresponding second portions of the retina of the eye 160 described above, which are respectively illuminated by the optical system 300 and imaged by the FD-OCT imaging system 320 according to the example embodiment. These figures show a region 600 of the retina of eye 160 which corresponds to the field of view of the FD-OCT imaging system 130. That is, the second portion of the eye 160 (at which the OCT data 135 is acquired) can be at any location within the region 600 to which the scanning system 300 can direct the sample OCT light Lₒ. As the optical system 300 shares the scanning system 310 with the FD-OCT imaging system 320, the first portion of the eye 160 can also be at any location within the region 600 to which the scanning system 300 can direct the light Lₛ. Note that the light Lₛ may be generated by the light source 301 as a light beam which may be of differing diameters, which may result in the differing sizes (widths) of the respective first portions as shown in each of Figures 6A, 6B and 6C. Different beam sizes (e.g. diameters) for the light Iₛ may be achieved using differing illumination source apertures, for example.

In particular, where the optical system 300 shares the scanning system 310 with the FD-OCT imaging system 320, the radius of the light beam (as the light Lₛ generated by the light source 301) may be set such that the light beam illuminates the whole of the second portion of the eye 160 while the light beam is at each scan location within the second portion of the eye 160. For example, where the second portion is a straight portion corresponding to a straight B-scan, the radius of the light beam may be set so as to illuminate the whole straight portion when the light beam is at the first scan location (corresponding to the first A-scan of the straight B-scan) and at each subsequent scan location. This arrangement prevents the 'flickering' which may be perceived by a part of the retina due to the alternate illumination and lack thereof of the part of the retina by the light beam as it moves between scan locations, which may improve the quality of the generated ORG data.

Figure 6A shows a circular first portion 610 as an example of the first portions of the retina referred to in the description of process S55 of Figure 5A, and a second portion 611, corresponding to an A-scan, as an example of the second portions of the retina referred to in the description of process S56. In process S56 of Figure 5A, the controller 140 controls the FD-OCT imaging system 320 to acquire OCT data in the form of a temporal sequence of A-scans of the second portion 611, by rotating the first scanning element 312 and the second scanning element 314 in the scanning system 310 to a first pair of respective angular positions such that each of the A-scans is acquired at the location of the second portion 611. At the aforementioned first time, the controller 140 performs process S55 of using an indicator of the N second indicators 142 to control the optical system 300 to apply the optical stimulus to the circularfirst portion 610 via the generation of the light Lₛ by the light source 301 (the diameter of the circular first portion being the diameter of the light Lₛ incident upon the retina). As the circular first portion 610 is concentric with the second portion 611, the first scanning element 312 and the second scanning element 314 remain at the first pair of respective angular positions during the acquisition of the ORG data 150, thus simplifying this process.

Figure 6B shows a hollow circular first portion 620 as an example of the first portions of the retina referred to in the description of process S55 of Figure 5A, and a second portion 621, corresponding to a circular B-scan, as an example of the second portions of the retina referred to in the description of process S56 of Figure 5A. In this case, the controller 140 controls the FD-OCT imaging system 320 to acquire OCT data in the form of a temporal sequence of the circular B-scans of the second portion 621, by rotating the first scanning element 312 and the second scanning element 314 in the scanning system 310 to perform a two-dimensional point scan using a circular scan pattern such that each of the circular B-scans is of the second portion 621. At the aforementioned first time, the controller 140 performs process S55 of using an indicator of the N second indicators 142 to control the optical system 300 to apply the optical stimulus to the hollow circular first portion 620 by rotating the first scanning element 312 and the second scanning element 314 in the scanning system 310 while the light Lₛ is being generated by the light source 301 (the thickness of the hollow circular first portion 620 being the diameter of the light Lₛ incident upon the retina). As the hollow circular first portion 620 and the second portion 621 following the same scan pattern, the same rotation of the first scanning element 312 and the second scanning element 314 (coordinated by the controller 140) can be used to provide the optical stimulus and acquire the OCT data 135, which simplifies operation of the FD-OCT apparatus 100.

Figure 6C shows a straight first portion 630 as an example of the first portions of the retina referred to in the description of process S55 of Figure 5A, and a straight second portion 631, corresponding to a straight B-scan, as an example of the second portions of the retina referred to in the description of process S56 of Figure 5A. The controller 140 performs processes S55 and S56 of Figure 5A in a similar manner to as described above with reference to Figure 6B (the thickness of the straight first portion 630 being the diameter of the light Lₛ incident upon the retina), except that the predetermined scan pattern is a unidirectional scan along a single scan axis (for the single straight B-scan). The straight first portion 630 and the straight second portion 631 run along a common straight line such that the same rotation of the first scanning element 312 and/or the second scanning element 314 (controlled by the controller 140) can be used to provide the optical stimulus and acquire the OCT data 135, which simplifies operation of the FD-OCT apparatus 100.

Although the first portions and the corresponding second portions in Figures 6A, 6B and 6C have been described as concentric, or running along a common line, this is not necessarily the case, and any other kind of scan pattern may be used. Further, although the circular first portion 610, the hollow circular first portion 620 and the straight first portion 630 have been described as having respective diameters or thicknesses corresponding to the diameter of the light Lₛ incident upon the retina, the diameter of the light Lₛ incident upon the retina may alternatively be smaller than the intended width of the first portion, and the controller 140 may coordinate the rotation of the first scanning element 312 and the second scanning element 314 to progressively illuminate the whole of each first portion.

Further, although the first portions are shown to be larger than the corresponding second portions in Figures 6A, 6B and 6C, this need not be the case. For example, an illumination source aperture of the light source 301 may be selected to provide light Lₛ of a diameter smaller than that of the sample OCT light Lₒ, or the second portion may extend outside of the first portion of the eye 160 by the controller correspondingly controlling the rotation of one of or both the first scanning element 312 and the second scanning element 314.

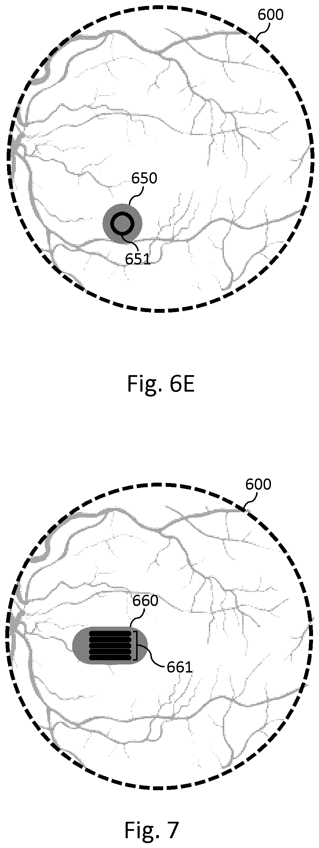

Figures 6D and 6E are schematic illustrations of example first portions of the retina and corresponding example second portions of the retina within the region 600, which are respectively illuminated by the optical system 360 and imaged by the FD-OCT imaging system 361 of Figure 3E.

Figure 6D shows a circular first portion 640 as an example of the first portions of the retina referred to in the description of process 555, and a second portion 641, corresponding to an A-scan, as an example of the second portions of the retina referred to in the description of process S56 of Figure 5A. As shown in Figure 6D, the second portion 641 is not concentric with the circular first portion 640, and this is enabled by the independent control of the location of the optical stimulus. In contrast to Figures 6A to 6C, the optical system 360 may apply the optical stimulus independently of where the sample OCT beam Lₒ is directed onto the retina, such that the optical stimulus can be applied to any required portion of the retina without interrupting the acquisition of the temporal sequence of OCT images.

Figure 6E shows a circular first portion 650 as an example of the first portions of the retina referred to in the description of process S55 of Figure 5A, and a second portion 651, corresponding to a circular B-scan (acquired using a circular scan as the predetermined scan pattern), as an example of the second portions of the retina referred to in the description of process S56 of Figure 5A. These geometries can likewise be achieved by the independent control of where the optical stimulus is applied to the retina and where the OCT data are acquired from the retina.

Figure 7 is a schematic illustration of a straight first portion 660 as an example of the first portions of the retina referred to in the description of process S55 of Figure 5A, and a second portion 661, corresponding to a plurality of parallel straight B-scans forming a C-scan (acquired using a raster scan as the predetermined scan pattern), as an example of the second portions of the retina referred to in the description of process S56 of Figure 5A, where the controller 140 controls the optical system 340 to provide the optical stimulus, and FD-OCT imaging system 341 to acquire the OCT data 135. In this case, the optical stimulus is controllable independently from the sample OCT beam Lₒ in the first direction, and the direction opposite to the first direction, which is perpendicular to the straight B-scans. This allows the optical stimulus to be applied to the straight first portion 630 without affecting the movement of the sample OCT beam Lₒ in the second direction between the intended location of each of the plurality of parallel straight B-scans. Note that similar C-scans may be obtained both by using the optical system 360 and FD-OCT imaging system 361, at the expense of increased system complexity, and using the optical system 300 and FD-OCT imaging system 320 at the expense of interrupting the movement of the sample OCT beam Lₒ in the second direction between the intended location of each of the plurality of parallel straight B-scans.

The target locations on the retina that are indicated by the second indicators 142, and therefore the first portions of the retina (to which the optical stimulus is applied) and the second portions of the retina (from which the OCT data 135 is acquired) may be distributed across the retina in any suitable or desirable way, in accordance with one or more scan patterns that may be used by the controller 140 to steer the optical stimulus and sample OCT light Lₒ across the retina as described above. Each scan pattern may be stored in association with a corresponding value of the first indicator 141, and preferably also in association with a scan pattern identifier so that the user is able to select (by any suitable input means, for example as described herein) from two or more scan patterns that are available for each value for the first indicator 141.

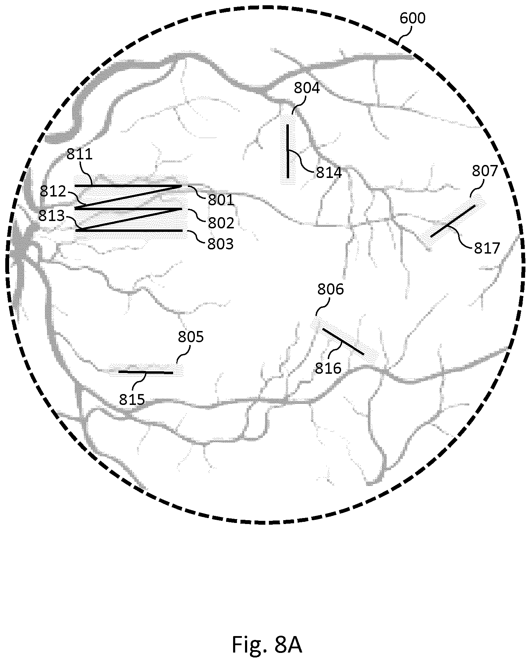

Figures 8A to 8E are schematic illustrations of example first portions of the retina and example second portions of the retina referred to in the description of processes S55 and S56 of Figure 5A, which are respectively illuminated by any of the implementations of the optical system 120, and imaged by any of the implementations of the FD-OCT imaging system 130 according to the example embodiment (e.g. the optical system 300 and the FD-OCT imaging system 320), unless otherwise stated. These figures show the region 600 of the eye 160 corresponding to the field of view of the FD-OCT imaging system 130 shown in Figures 6A to 6E and 7.

Figure 8A shows a plurality of first portions 801 to 807 as an example of the first portions in process S55 of Figure 5A, and a plurality of second portions 811 to 817 as an example of the second portions in process S56 of Figure 5A, in the ORG capture session. The controller 140 performs processes S55 and S56 to stimulate the first portion and acquire OCT data 135 from the corresponding second portion for each pair of first and second portions 801 and 811, 802 and 812, 803 and 813, etc. that are disposed at different locations on the retina, in turn, in the manner described above in relation to the straight first portion 630 and the second portion 631 in Figure 6C, for example.

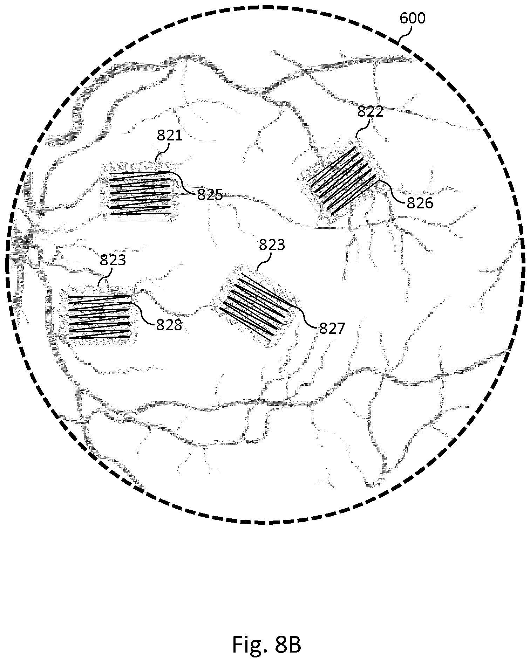

Figure 8B shows a plurality of first portions 821 to 824 as an example of the first portions in process S55 of Figure 5A, and a plurality of second portions 825 to 824 as an example of the second portions in process S56 of Figure 5A, in the ORG capture session. The controller 140 performs processes S55 and S56 to stimulate the first portion and acquire OCT data 135 from the corresponding second portion for each pair of first and second portions 821 and 825, 822 and 826, 823 and 827, etc. that are disposed at different locations on the retina, in turn, in the manner described above in relation to the straight first portion 660 and the second portion 661 in Figure 7, for example.

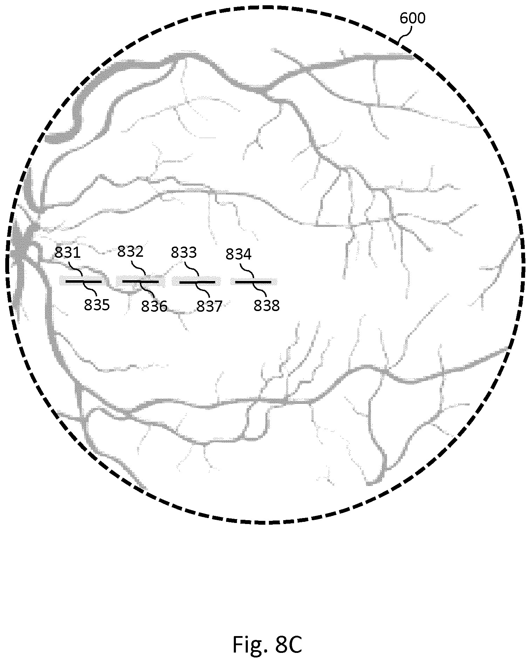

Figure 8C shows a plurality of first portions 831 to 834 as an example of the first portions in process S55 of Figure 5A, and a plurality of second portions 835 to 838 as an example of the second portions in process S56 of Figure 5A, in the ORG capture session, which may be used to perform a radial trend analysis of the eye 160 via analysing the variation of the retinal response of the eye 160 with eccentricity of the eye 160 (e.g. from the periphery of the eye 160 to the fovea of the eye 160). The controller 140 performs processes S55 and S56 to stimulate the first portion and acquire OCT data 135 from the corresponding second portion for each pair of first and second portions 831 and 835, 832 and 836, 833 and 837, etc. that are disposed at different locations along a line on the retina, in turn, in the manner described with reference to the straight first portion 630 and the second portion 631 in Figure 6C, for example.

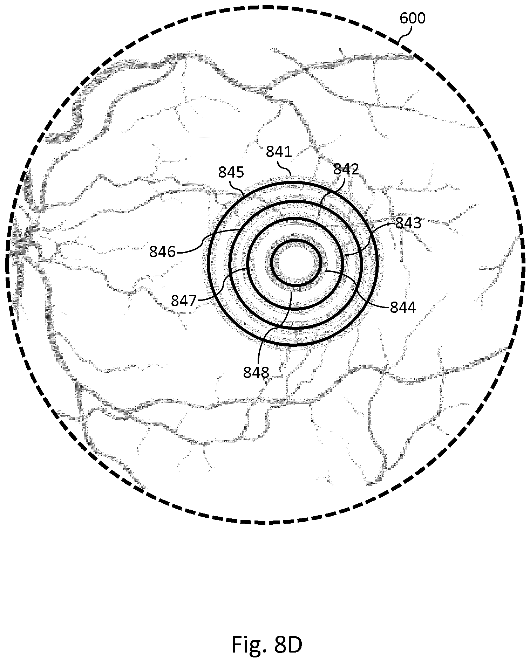

Figure 8D shows a plurality of first portions 841 to 844 as an example of the first portions in process S55 in Figure 5A, and a plurality of second portions 845 to 848 as an example of the second portions in process S56 of Figure 5A, in the ORG capture session, which may be also used to perform a radial trend analysis of the eye 160, where the variation of the retinal response of the eye 160 with eccentricity of the eye 160 is analysed. To achieve this, each of the first and second portions may form concentric ring-like regions that are centred on the fovea of the eye 160, which correspond to different degrees of eccentricity of the eye 160. The controller 140 performs processes S55 and S56 to stimulate the first portion and acquire OCT data 135 from the corresponding second portion for each pair of first and second portions 841 and 845, 842 and 846, 843 and 847, etc. that are disposed at different distances from the fovea, in turn, in the manner described above with reference to the hollow circular first portion 620 and the second portion 621 in Figure 6B.

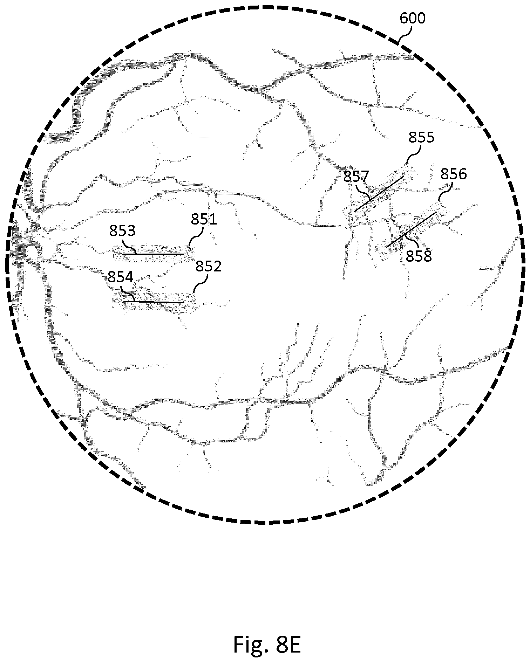

Figure 8E shows a plurality of first portions 851, 852, 855, 856 as an example of the first portions in process S55 of Figure 5A, and a plurality of second portions 853, 854, 857, 858 as an example of the second portions in process S56 of Figure 5A, in the ORG capture session. The controller 140 performs processes S55 and S56 to stimulate the first portion and acquire OCT data 135 from the corresponding second portion for each pair of first and second portions 851 and 853, 855 and 857, etc. that are disposed at different locations on the retina, in turn, in the manner described with reference to the straight first portion 630 and the second portion 631 in Figure 6C, for example. In this case, the controller 140 controls the optical system 120 to apply a first optical stimulus to the first portions 851 and 852 on the retina, and to applies a second optical stimulus to the second portions 855 and 856, wherein the first optical stimulus is different to the second optical stimulus (e.g. by the light source of the optical system 120 generating light at two different wavelengths or the optical system 120 comprising two differing light sources arranged to generate light at different wavelengths). Although two differing optical stimuli are described, the optical system 120 is not so limited and may be operable to generate further differing optical stimuli (e.g. as described with reference to the light source 301 of the optical system 300 in Figure 3A).

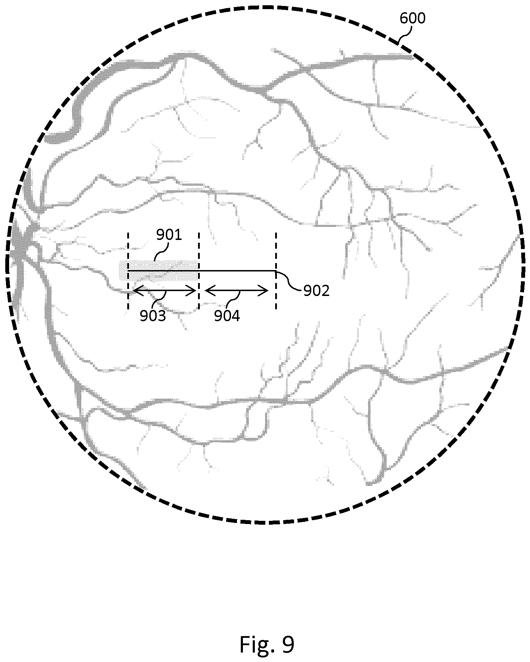

Figure 9 shows a first portion 901 and a second portion 902 in the region 800, which may respectively be examples of the first portion in process S55 of Figure 5A and the second portion in process S56 of Figure 5A. A stimulus applied to the first portion 901 stimulates a first part 903 of the second portion 902 but does not stimulate a second part 904 of the second portion 902. Thus, the second part 904 provides a baseline unstimulated region which can be used for comparison and/or normalisation purposes. This technique of partially stimulating the second portion (by performing a temporal modulation of a signal used to generate the optical stimulus) may also be used in any of the other examples describe above with reference to Figures 8A to 8E. Further, although this technique has been described in relation to a straight first portion 630 and a straight second portion 631, it will be appreciated that this technique is not so limited, and the principles described may be applied to other geometries of first and second portions of the retina.

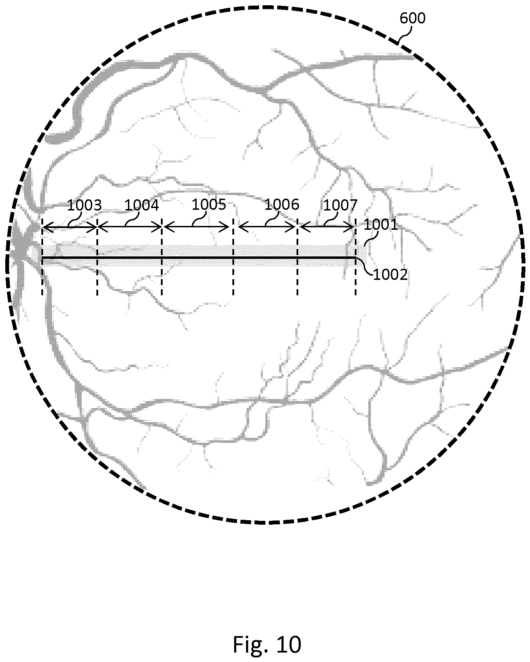

In some cases, the controller 140 may advantageously split the processing of the OCT data 135, to generate the ORG data 150, into smaller steps. Figure 10 shows an example first portion 1001 and an example second portion 1002 in the region 800 which may, respectively, provide examples of a first portion of the first portions in process S55 and a second portion of the second portions in process S56 of Figure 5A. As shown in Figure 10, the OCT data of the second portion 1002 may be divided into subsets of the OCT data respectively corresponding to regions 1003 to 1007, and each of the subsets of the OCT data may be separately processed using the above-described techniques to generate the ORG data 150 for each of the parts 1003 to 1007 on the retina of the eye 160. For example, in the velocity-based ORG technique described above, the temporal sequence of partial B-scans acquired from each region of the regions 1003 to 1007 may be used as the temporal sequence of B-scans in this algorithm to generate ORG at the respective location of the temporal sequence of the partial B-scans. This technique of splitting up the processing of the second portion may also be performed for at least some of the remaining portions of the plurality of second portions (if any). Further, while this technique has been shown and described in relation to a straight first portion 1001 and a straight second portion 1002, it will be appreciated that this technique is not so limited, and the principles described may be applied to other geometries of first and second portions of the retina.

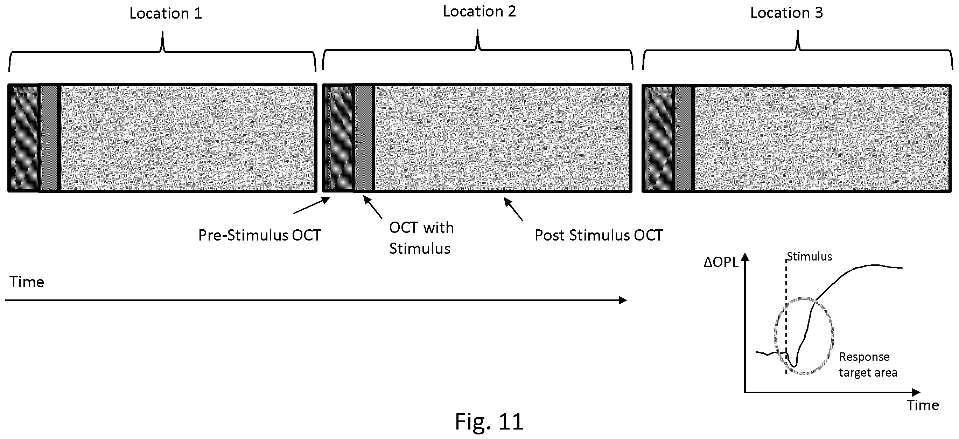

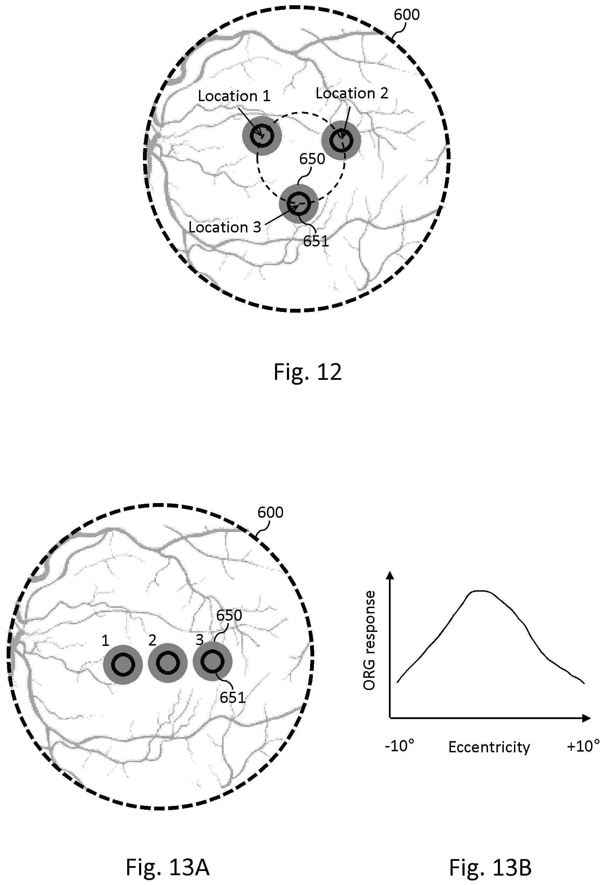

Figure 11 is a schematic illustration of a schedule for successively acquiring sets of circular B-scans of the kind described above in relation to Figure 6E, at three different locations on the retina as shown in Figure 12. Naturally, there may be fewer or more than the shown number of target locations in this example and in the other examples described below with reference to Figures 13 to 16. The ORG data 150 acquired from the B-scans in the present example relates to the fast response and an initial part of the slow response of the retina, as shown in the inset in Figure 11, where the change in optical path length (ΔOPL) of the OS is plotted as a function of time. In accordance with the schedule of Figure 11, 10 B-scans of pre-stimulus OCT data are acquired, followed by five B-scans of OCT data while the stimulus is being applied, followed by 50 B-scans of post-stimulus OCT data, all for Location 1 shown in Figure 12. This process is repeated for different Location 2 and different Location 3. In this example, a sequence of B-scans is acquired over 70 ms at each of Location 1, Location 2 and Location 3 on the retina, as shown in Figure 12, resulting in a total scan time which is a little over 210 ms (owing to delays associated with the transition of the scan location from Location 1 to Location 2, and from Location 2 to Location 3). It should be noted, however, that the respective numbers of pre-stimulus B-scans, stimulus B-scans and/or post-stimulus B-scans may vary among the locations on the retina in general, and the sequences of B-scans may, in general, be acquired on different timescales at Location 1, Location 2 and Location 3. For the slow retinal response, it is not possible to acquire further reliable ORG data 150 by repeating the scan sequence defined by the schedule in Figure 11 without waiting, between each repetition of the scan sequence, for a recovery time (of approximately 5 minutes) necessary for the photoreceptors in the retina to recover to their baseline length in a dark environment from the applied optical stimuli.

Figure 12 is a schematic illustration of an arrangement, along a circle centred on the fovea of the eye 160, of example first portions 650 and second portions 651 of the retina that are illuminated in the performance of processes S55 and S56 in Figure 5A, using the optical system 360 and the FD-OCT imaging system 361 in Figure 3E and in accordance with the schedule in Figure 11. The first and corresponding second portions, 650 and 651, are distributed, in accordance with the second indicators 142, at respective target locations on the circle centred on the fovea. The ORG data 150 acquired from these locations can be expected to be similar and thus suitable for averaging and/or comparison. Although the OCT data 135 takes the form of circular B-scans in the present example, the OCT data 135 may take other forms, for example straight-line B-scans or volume scans (C-scans).

Figure 13A is a schematic illustration of an alternative arrangement, along a straight line passing through the fovea, of example first portions 650 and second portions 651 of the retina that are illuminated in the performance of processes S55 and S56 in Figure 5A, using the optical system 360 and the FD-OCT imaging system 361 in Figure 3E and in accordance with the schedule in Figure 11. The circular B-scans are acquired in the sequence (1, 2, 3) shown in Figure 13A. The resulting ORG data 150 is thus acquired across different eccentricity, allowing the ORG response (e.g. in terms of a change in optical path length) to be plotted as a function of eccentricity. As an example, Figure 13B provides a schematic illustration of an example ORG response plotted as a function of eccentricity, which may be acquired using a linear arrangement of first and second portions of the kind illustrated in Figure 13A. Such plots may be useful in an investigation of diseases such as retinitis pigmentosa, where the disease propagates from the periphery over time and its progression is often characterised in terms of "diseased", "transition" and "healthy" regions going from periphery to the central pole. A plot of ORG response vs. eccentricity plot could be applied to assess disease progression or severity.

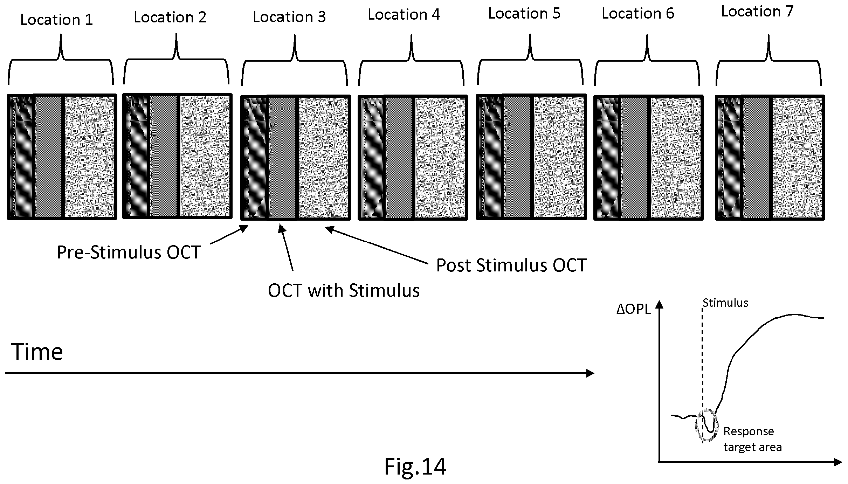

Figure 14 is a schematic illustration of another schedule for successively acquiring sets of circular B-scans of the kind described herein with reference to Figure 6E, at different locations on the retina, to acquire ORG data 150 relating only to the fast response of the retina shown in the inset of Figure 14. In this example, two B-scans of pre-stimulus OCT data are acquired, followed by two B-scans of OCT data while the stimulus is being applied, followed by 10 B-scans of post-stimulus OCT data, all for Location 1. This process is then repeated for different Locations 2 to 7. In this example, a sequence of B-scans is acquired over 20 ms at each of Location 1 to Location 7 on the retina, resulting in a total scan time which is a little over 140 ms (owing to delays associated with the transition of the scan location from Location 1 to Location 2, from Location 2 to Location 3, etc.).

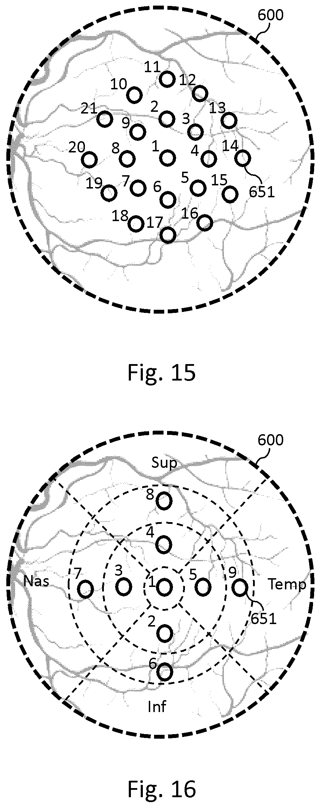

Figure 15 is a schematic illustration of 22 example second portions of the retina scanned in the performance of process S56 in Figure 5A, using the FD-OCT imaging system 361 in Figure 3E and a schedule which is similar to the fast-response schedule of Figure 14 but targets 21 locations on the retina instead of seven. For clarity, the first portions 650 are not shown in Figure 15. In the example of Figure 15, the sets of circular B-scans are acquired in the sequence shown (1, 2, 3...21), such that their locations on the retina lie on concentric circles that are centred on the fovea for eccentricity commonality, and preferably cover most or all of the macula.

Figure 16 is a schematic illustration of an alternative arrangement of none example second portions of the retina, in corresponding portions of an Early Treatment Diabetic Retinopathy Study (ETDRS) grid, that are scanned in the performance of process S56 in Figure 5A using the FD-OCT imaging system 361 of Figure 3E and in accordance with a schedule which is similar to the fast-response schedule of Figure 14 but targets nine locations on the retina instead of seven. For clarity, the first portions 650 are not shown in Figure 16. The ORG data 150 acquired from the second portions 651 in Figure 16 can be used to assess retinal health by area, and may be compared to the results of other investigations, such as microperimetry tests and retinal thickness measurements.

Various further variations and modifications may be made to the example embodiments described above.

For example, although some of the examples of the optical system 120 described above employ scanning elements 363 and 364, or a single two-dimensional scanner 373, to direct the light Lₛ to the retina of the eye 160 and vary the location on the retina of the eye 160 at which the light Lₛ is to be applied, one or more scanning elements of a different kind may instead be used for this purpose.

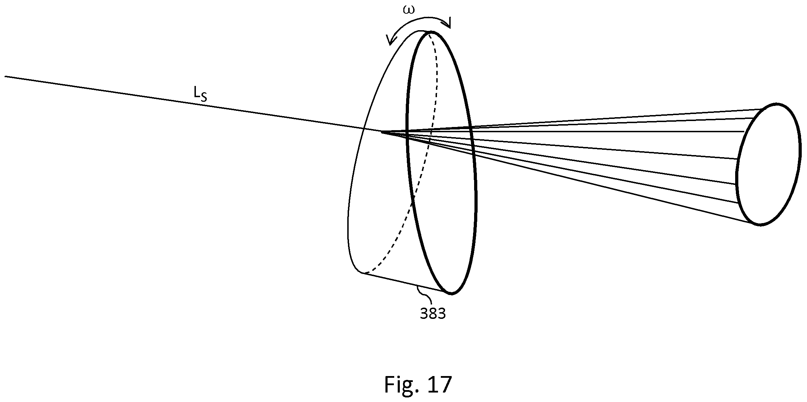

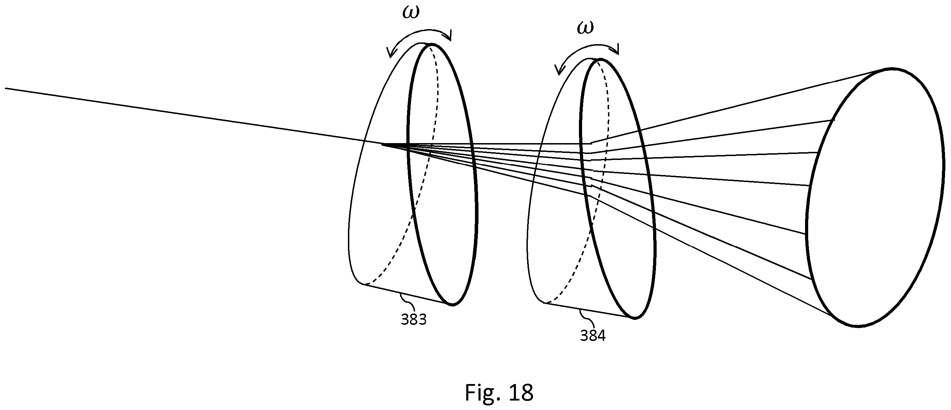

For example, one or more refractive scanning elements may be used instead of one or more refractive elements. More particularly, a beam of the light Lₛ may be arranged to impinge on a scanning element in the form of a rotatable wedged prism 383, as illustrated schematically in Figure 17, and the wedged prism 383 may be driven (by a galvanometer or other rotational drive mechanism, for example) to rotate about a rotational axis which may be aligned with the propagation direction of the beam of light Lₛ, and thus deflect the beam of light Lₛ to propagate in a direction which is determined by the rotational position of the wedged prism 383. In some variants, a second wedged prism 384 may be added to provide a so-called Risley prism scanner, wherein the two wedged prisms 383 and 384 are independently rotatable about respective rotational axes that may be aligned (parallel) to each other and in some cases coincide, as illustrated schematically in Figure 18. Such a configuration of two or more wedged prisms may allow the beam of light Lₛ to be steered over a greater variety of locations on the retina than a single wedged prism.

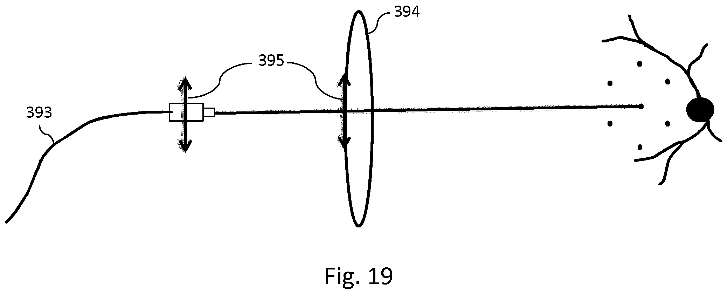

The optical system 120 may be configured to vary the location on the retina at which the optical stimulus is to be applied in yet further ways. For example, instead of using scanning elements 363 and 364, the single two-dimensional scanner 373 or the one or more wedged prisms 383, 384 described above, the optical system 120 may be arranged to project the light Lₛ towards the retina of the eye 160 via an optical fibre 393 and at least one lens 394 that is arranged to focus the light Lₛ from the optical fibre 393, wherein an end of the optical fibre 393, from which the light Lₛ emerges, is movable relative to the at least one lens 394 in a direction which may be normal to the direction of propagation of the light Lₛ between the end of the optical fibre 393 and the at least one lens 394. Such an arrangement is illustrated schematically in Figure 19. The location on the retina at which the optical stimulus is to be applied may thus be varied by a translation of the end of the optical fibre 393 along the directions 395 in Figure 19 while the at least one lens 394 remains stationary, by a translation of the at least one lens 394 along the directions 395 while the end of the optical fibre 393 remains stationary, or by any other relative movement between the end of the optical fibre 393 and the at least one lens 394 along the directions 395 in Figure 19.

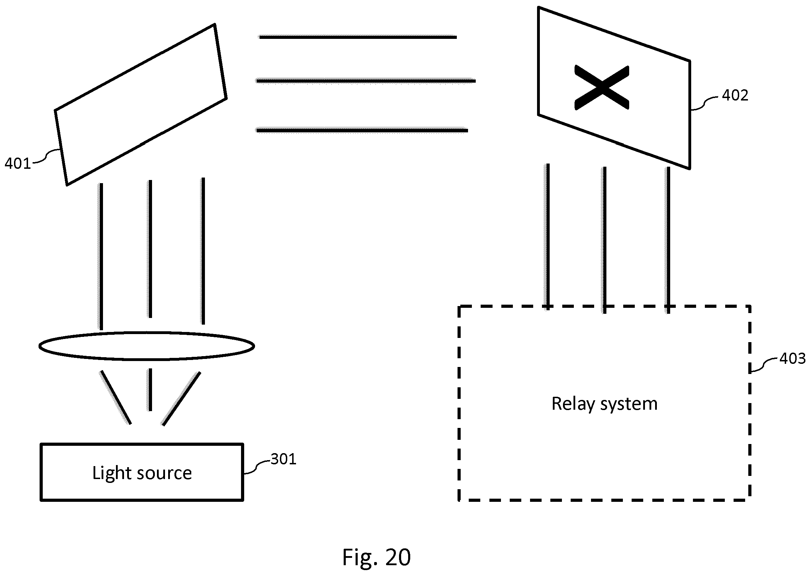

In relation to the example embodiments comprising a dynamic amplitude mask described above it is noted that, regardless of the form in which it is implemented, the dynamic amplitude mask may be sized so as to allow the unmasked portion thereof to illuminate any location on the retina of the eye 160. However, there is a risk that some or all elements of the dynamic amplitude mask (e.g. some or all of the micromirrors, in case the dynamic amplitude mask is provided in the form of a DMD, as described above) may fail and consequently cause the eye 160 to be illuminated with an optical power which may exceed safe limits. To address this issue, in some example embodiments, the dynamic amplitude mask is sized (made small enough) so that the optical power it directs towards the eye 160 in the event of a total failure of the dynamic amplitude mask (wherein substantially all the light incident on the dynamic amplitude mask is directed towards the eye 160) remains under a predetermined threshold level which is safe for the eye 160. In this case, as illustrated in Figure 20, the limitation in the size of the dynamic amplitude mask 401 may be compensated for by providing a galvanometer scanner or other beam steering mechanism 402 in the optical pathway between the dynamic amplitude mask 401 and the eye 160, which beam steering mechanism 402 is controllable by the controller 140 to steer the light from the dynamic amplitude mask 401 towards the target location on the retina indicated by the indicator 142. The location on the retina at which the optical stimulus is to be applied may thus be adjusted partly by the configuration of the unmasked portion of the dynamic amplitude mask 401, and partly by the beam steering mechanism 402, both of which may be controlled by the controller 140. The relay system 403 shown in Figure 20 encompasses mirror-based relay systems of the kind described above with reference to Figures 3A and 3C (which comprise curved mirrors 313 and 315), as well as lens-based relay systems of the kind described above with reference to Figures 3B and 3D to 3F (which comprise lens relay 334).

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as functions of the controller 140, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to optical disks and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the controller 140 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents. It is also to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. An optical coherence tomography, OCT, apparatus (100) arranged to acquire optoretinography, ORG, data (150) that is indicative of a physiological response of a retina of an eye (160) of a subject to an optical stimulus, the OCT apparatus (100) comprising:
an optical system (120) operable to apply the optical stimulus to the retina such that an illumination of the retina by the optical stimulus is confined to a portion of the retina, the optical system (120) being controllable to vary a location on the retina at which the optical stimulus is to be applied;
an OCT imaging system (130) operable to acquire OCT data (135) by imaging a portion of the retina of the eye (160); and
a controller (140) arranged to:
acquire (S51) a first indicator (141) which is indicative of a duration of the physiological response indicated by ORG data that is to be acquired by the OCT apparatus (100);
acquire (S54) a number, N, of second indicators (142), each second indicator being indicative of a respective target location on the retina at which the optical stimulus is to be applied by the optical system (120), wherein N is dependent on the first indicator (141) such that N decreases as the duration indicated by the first indicator (141) increases;
use (S55) the second indicators (142) to control the optical system (120) to apply the optical stimulus to respective first portions of the retina at the respective target locations;
control (S56) the OCT imaging system (130) to acquire, for each of the first portions of the retina, respective OCT data (135) of a respective second portion of N second portions of the retina over the duration indicated by the first indicator (141), wherein at least a part of the respective second portion of the retina is disposed in relation to the respective first portion of the retina so as to be stimulated by the applied optical stimulus during acquisition of at least some of the respective OCT data (135); and
process (S57) the respective OCT data (135) of each second portion of the retina to generate respective ORG data (150) indicative of a respective physiological response of the second portion of the retina to the optical stimulus applied to the corresponding first portion of the retina.

2. The OCT apparatus (100) according to Claim 1, wherein the controller (140) is arranged to acquire the first indicator (141) by selecting a value from a group of values comprising:
a first value indicative of a duration less than 20 ms of the physiological response indicated by ORG data (150) that is to be acquired by the OCT apparatus (100); and
a second value indicative of a duration greater than 20 ms of the physiological response indicated by ORG data (150) that is to be acquired by the OCT apparatus (100).

3. The OCT apparatus (100) according to Claim 1 or Clam 2, wherein the N second indicators (142) are indicative of respective target locations on the retina that are one of:
distributed around a circle centred on a fovea of the eye (160), at which target locations the optical stimulus is to be applied by the optical system (120);
located within respective grid cells of an Early Treatment Diabetic Retinopathy Study, ETDRS, grid centred on a fovea of the eye (160), at which target locations the optical stimulus is to be applied by the optical system (120); and
distributed along a straight line passing through a fovea of the eye (160), at which target locations the optical stimulus is to be applied by the optical system (120).

4. The OCT apparatus (100) according to any preceding claim, wherein
the optical system (120, 300) comprises a light source (301) arranged to generate light which provides the optical stimulus, and one or more scanning elements (312, 314; 363, 364) arranged to direct the light to the retina, and
the controller (140) is arranged to use the N second indicators (142) to control the one or more scanning elements (312, 314; 363, 364) to direct the light to each of the first portions of the retina which is at the respective target location.

5. The OCT apparatus (100) according to Claim 4, wherein
the OCT imaging system (130, 320) comprises:
an interferometer (322) having a sample arm (324) and a reference arm (325); and
a detector (323) arranged to detect an interference between sample OCT light (Lₒ) propagating along the sample arm (324) after having been scattered from the retina, and reference OCT light (Lᵣ) propagating along the reference arm (325), and
at least one of the one or more scanning elements (312, 314) is further arranged to direct the sample OCT light (Lₒ) toward each of the N second portions of the retina, and the sample OCT light (Lₒ) scattered from each of the N second portions of the retina toward the detector (323).

6. The OCT apparatus (100) according to Claim 4, wherein
the OCT imaging system (120, 361) comprises:
an interferometer (322) having a sample arm (324) and a reference arm (325);
one or more scanning elements (312, 314); and
a detector (323) arranged to detect an interference between sample OCT light (Lₒ) propagating along the sample arm (324) after having been scattered from the retina, and reference OCT light (Lᵣ) propagating along the reference arm (325),
wherein the one or more scanning elements (312, 314) are arranged to direct the sample OCT light (Lₒ) toward each of the N second portions of the retina, and the sample OCT light (Lₒ) scattered from each of the N second portions of the retina toward the detector (323), and
the one or more scanning elements (363, 364) of the optical system (120, 360) are different from the one or more scanning elements (312, 314) of the OCT imaging system (120, 361).

7. The OCT apparatus (100) according to Claim 6, wherein the controller (140) is arranged to use the N second indicators (142) to control the one or more scanning elements (363, 364) of the optical system (130, 361) independently from the one or more scanning elements (312, 314) of the OCT imaging system (120, 360).

8. The OCT apparatus (100) according to any preceding claim, wherein
the controller (140) stores a third indicator (143) which is indicative of a period of time over which the optical system (120) is to apply the optical stimulus to respective first portions of the retina and the OCT imaging system (130) is to acquire, for each of the first portions of the retina, the respective OCT data (135) of the respective second portion of the retina over the duration indicated by the first indicator (141), and
the controller (140) is arranged to determine, based on the first indicator (141) and the third indicator (143), the number, N, of second indicators (142) to be acquired such that, within the period of time indicated by the third indicator (143):
the controller (140) uses the N second indicators (142) to control the optical system (120) to apply the optical stimulus to the respective first portions of the retina; and
the controller (140) controls the OCT imaging system (130) to acquire the respective OCT data (135) for each of the first portions of the retina.

9. The OCT apparatus (100) according to Claim 8, wherein the controller (140) is arranged to update the third indicator (143) based on a size of the pupil of the eye (160) such that the period of time indicated by the third indicator (143) increases as the size of the pupil increases.

10. The OCT apparatus (100) according to Claim 8 or Claim 9, wherein the period of time indicated by the third indicator (143) does not exceed 300 ms.

11. A computer-implemented method of controlling an optical coherence tomography, OCT, apparatus (100) to acquire optoretinography, ORG, data (150) that is indicative of a physiological response of a retina of an eye (160) to an optical stimulus, the OCT apparatus (100) comprising:
an optical system (120) operable to apply the optical stimulus to the retina such that an illumination of the retina by the optical stimulus is confined to a portion of the retina, the optical system (120) being controllable to vary a location on the retina at which the optical stimulus is to be applied; and
an OCT imaging system (130) operable to acquire OCT data (135) by imaging a portion of the retina of the eye (160),
the method comprising:
acquiring (S51) a first indicator (141) which is indicative of a duration of the physiological response indicated by ORG data that is to be acquired by the OCT apparatus (100);
acquiring (S54) a number, N, of second indicators (142), each second indicator being indicative of a respective target location on the retina at which the optical stimulus is to be applied by the optical system (120), wherein N is dependent on the first indicator (141) such that N decreases as the duration indicated by the first indicator (141) increases;
using(S55) the second indicators (142) to control the optical system (120) to apply the optical stimulus to respective first portions of the retina at the respective target locations;
controlling (S56) the OCT imaging system (130) to acquire, for each of the first portions of the retina, respective OCT data (135) of a respective second portion of N second portions of the retina over the duration indicated by the first indicator (141), wherein at least a part of the respective second portion of the retina is disposed in relation to the respective first portion of the retina so as to be stimulated by the applied optical stimulus during acquisition of at least some of the respective OCT data (135); and
processing (S57) the respective OCT data (135) of each second portion of the retina to generate respective ORG data (150) indicative of a respective physiological response of the second portion of the retina to the optical stimulus applied to the corresponding first portion of the retina.

12. The computer-implemented method according to Claim 11, wherein the first indicator (141) is acquired by selecting a value from a group of values comprising:
a first value indicative of a duration less than 20 ms of the physiological response indicated by ORG data (150) that is to be acquired by the OCT apparatus (100); and
a second value indicative of a duration greater than 20 ms of the physiological response indicated by ORG data (150) that is to be acquired by the OCT apparatus (100).

13. The computer-implemented method according to Claim 11 or Claim 12,further comprising:
storing (S52) a third indicator (143) which is indicative of a period of time over which the optical system (120) is to apply the optical stimulus to respective first portions of the retina and the OCT imaging system (130) is to acquire, for each of the first portions of the retina, the respective OCT data (135) of the respective second portion of the retina over the duration indicated by the first indicator (141); and
determining (S53), based on the first indicator (141) and the third indicator (143), the number, N, of second indicators (142) to be acquired such that, within the period of time indicated by the third indicator (143):
the N second indicators (142) are used to control the optical system (120) to apply the optical stimulus to the respective first portions of the retina; and
the OCT imaging system (130) is controlled to acquire the respective OCT data (135) for each of the first portions of the retina.

14. A computer program (445) comprising computer-readable instructions that, when executed by a processor (420) which is arranged to control the optical coherence tomography, OCT, apparatus (100) according to any of Claims 1 to 10, cause the processor (420) to control the OCT apparatus (100) in accordance with the method according to any of Claims 11 to 13.

15. A non-transitory computer-readable storage medium storing the computer program according to Claim 14.
